# EUROPEAN PATENT APPLICATION

(11) **EP 1 859 735 A1**
(43) Date of publication of application: **28.11.2007**
(21) Application number: 05785283.2
(22) Date of filing: 20.09.2005
(51) Int. Cl.: A61B 5/0245, A61B 5/145

(54) **BIOLOGICAL SIGNAL MEASUREMENT DEVICE, BIOLOGICAL SIGNAL MEASUREMENT METHOD, AND COMPUTER PROGRAM**

(30) Priority: 10.03.2005 JP 2005067947
(71) Applicant: Sharp Kabushiki Kaisha, Osaka-shi, Osaka 545-8522 (JP)
(72) Inventor: KAWAJIRI, Momoe, Osaka 544-0001 (JP); ASHIHARA, Kazuhiko, Kyoto 604-8404 (JP); ICHIKAWA, Yuhji, Nara 632-0046 (JP); NAKAGAWA, Katsuya, Kyoto 619-0223 (JP)
(74) Representative: Brown, Kenneth Richard
(86) International application number: PCT/JP2005/017290
(87) International publication number: WO 2006/095465

(57) **Abstract**

A biological signal measurement device includes: a circular or hyperbolic fitting section (101) to be worn on a part of a human body; a sensing section (103) which is provided to the fitting section (101), and senses a biological signal by being closely attached to a specific part of the human body; a measurement environment judging section for judging an environment for measuring the biological signal by using the sensing section (103); a measurement environment notifying section (104) for notifying a user of a judgment result, according to the result of judgment performed by the measurement environment judging section; and a measurement environment adjusting section (102) for adjusting the measurement environment of the sensing section (103). Thus, it is possible to provide a human-wearable biological signal measurement device that allows easier maintenance of suitable measurement conditions.

## Description

### TECHNICAL FIELD

The present invention relates to a biological signal measurement device worn on a human body for measuring a pulse wave or blood oxygen saturation.

### BACKGROUND ART

In recent years, there has been a demand for devices that allow easier measurement of biological signals (e.g. pulsebeat or oxygen saturation) for home care or preventive care in everyday life. That is, there is a demand for devices capable of conducting such measurement unnoticeably, without any constraint. Today, as an example of a biological signal measurement device which does not get in the way of doing something and which is advantageous in terms of portability, there is a ring-type biological signal measurement device wearable on a finger. Various structures have been suggested for such a ring-type biological signal measurement device to reduce measurement errors attributed to shifting of a measuring section from a targeted portion of a finger or rotation of the measuring section about the finger, thereby enabling a highly accurate and stable signal measurement even if a user wears the device at all time and walk or run with the device.

First described is a method of measuring a pulse wave. A blood vessel expands and shrinks with pulsation of the heart. Accordingly, when light is cast from a light emitting element of the biological signal measurement device, and the quantity of light received at a light receiving element paired with the light emitting element is measured, the quantity of light received at the light receiving element varies with the movement of the blood vessel. The waveform of this variation is measured as a pulse wave. A particularly large pulse wave is measured by casting light on an artery. Needless to say that pulse rate can be also measured from the pulse wave.

Next described is a method of measuring blood oxygen saturation. Blood oxygen saturation is a percentage of haemoglobin bond with oxygen (oxyheamoglobin) in blood. When light is cast on blood, the oxyheamoglobin and heamoglobin without oxygen respectively absorb light of different wavelengths. Using this characteristic, blood oxygen saturation is calculated and measured by (i) casting light of different wavelengths from the light emitting element of the biological signal measurement device and (ii) measuring the quantity of light received by the light receiving element paired with the light receiving element. The blood oxygen saturation thus measured is used as an indicator for diagnosis of respiratory symptoms: e.g., early detection of high-altitude disease, and diagnosis of sleep apnea syndrome.

Here, the above-described human-wearable biological signal measurement device needs to meet the following requirements, for more accurately measuring the biological signals.
(1) To be capable of applying a necessary pressure for a measurement to a finger.
   For example, when measuring a pulsebeat, it is difficult to do so without an application of a pressure to a blood vessel, because of a small amount of constriction of the blood vessel associated with a decrease in the blood stream (flow of blood) or in the blood pressure. However, application of a suitable pressure to the blood vessel causes the blood vessel to constrict and expand by a larger amount in association with increase/ decrease of the blood stream or the blood pressure. This makes the measurement easier. Of course, too large a pressure to the blood vessel keeps the blood vessel from expanding, making the measurement difficult. Thus, for measuring biological signals at a finger or the like, it is preferable that a suitable pressure be applied to the blood vessel. Further, to perform more accurate measurement, a structure capable of applying a constant pressure is needed for stabilizing the signals.
(2) To prevent shifting of a sensing section from the measuring object (blood vessel) of the finger.
   Performing highly-accurate measurement unsurprisingly requires that the sensing section be kept from shifting from the measuring object (blood vessel).
   To meet the Requirement (2), Patent Citation 1 (Japanese Unexamined Patent Application No. 124436/1989 (Tokukaihei 1-124436) discloses an acceleration sphygmograph which displays whether or not detection conditions are suitable. This is for enabling a user to adjust how he/she inserts his/her fingertip into a sensing section. Specifically, light from a light source of the acceleration sphygmograph passes through a capillary bed in a fingertip, and the quantity of light passed through the finger tip is detected by the acceleration sphygmograph. Then, an indicator displays whether or not a suitable signal width is obtained. Thus, the user is able to know whether or not the fingertip is inserted into the measuring device for a proper measurement of the pulse wave.
   To meet the Requirement (1), Patent Citation 2 (Japanese Unexamined Patent Application No. 232929/1989 (Tokukaihei 1-232929) discloses a pulse wave detecting device for measuring a pressure pulse wave at an artery, and a method of obtaining a suitable size of pulse wave by controlling a press force applied by a detector of the pulse wave detecting device. Specifically, in the method, the pressing force of the detector is continuously varied, and is set at a pressing force that results in the biggest pressure pulse wave through the detector. Then, the detector is pressed with the pressing force. Thus, a suitable size of pulse wave is always detected.
   To meet the Requirements (1) and (2), Patent Citation 3 (Japanese Unexamined Patent Application No. 332840/1999 (Tokukaihei 11-332840) discloses a ring-shaped biological signal detection device having an adjustment structure (e.g. spring) which reduces an inner circumference of a fitting section of the ring. In this device, since the spring reduces the inner circumference of the fitting section of the ring, a finger is squeezed by the entire inner circumference of the ring at a constant force. In other words, a constant pressure is applied to the finger. Further, highly-accurate measurement is achieved by (i) defining, as an optimum pressure (squeezing force), a pressure which allows the most accurate measurement and (ii) setting a load of the spring so that the optimum pressure is applied to a finger. Further, with the spring structure, the inner circumference of the fitting section of the ring is reduced. Thus, the finger is squeezed while wearing the ring. This prevents the ring from shifting by rotating, and prevents a gap between a finger and a sensing section for measuring biological signals.
   Incidentally, it is preferable that the biological signal measurement device be such that wearing of it at all time does not cause discomfort, and that measurement is conducted as needed under suitable environment. Accordingly, in addition to the foregoing Requirements (1) and (2), there is another requirement as described below.
(3) To be capable of easily adjusting pressure and position of a sensing section to a favorable state and maintain them.

Since the biological signal measurement device is designed to be worn on a human body at all time, the position and pressure of the device may vary due to a motion in everyday activities or a change in the body conditions. On this account, the device needs to be such that the user is easily able to adjust how the device is worn (hereinafter, fitting state) to a favorable state, and that the favorable state is maintained as much as possible.

Here, the above-described technologies disclosed in Patent Citations 1 through 3 are not capable of meeting the Requirement (3).

Firstly, the technology disclosed in Patent Citation 1 is not something to be worn on a part of a human body. Therefore, the technology is not for enabling adjustment of the fitting state as required in Requirement (3). Specifically, when adjusting the state of inserting a user's fingertip into the sensing section, the user him/herself has to adjust the state of inserting the fingertip by making a movement of his/her body, every time the measurement is conducted. Further, in the technology disclosed in Patent Citation 1, the measuring object is a group of capillary vessels at a fingertip. Therefore, this technology does not meet the Requirement (2).

The technology of Patent Citation 3 is aiming at meeting the Requirement (2), by preventing shifting of a sensing section from a measuring object (blood vessel) due to rotation or the like by applying a squeezing force to the finger with a use of a spring. However, if the sensing section shifted from the measuring object due to a motion in everyday activities or a change in the body conditions, the technology does not meet the Requirement (3) which is to allow the user to easily adjust the fitting state to a favorable state.

Further, the technology of Patent Citation 3 does not allow adjustment of the fitting state. Therefore, even if the squeezing force is within a suitable range for measurement of biological signals, the force is not necessarily suitable for wearing the device in everyday life without discomfort. The force may even cause a blood congestion in the finger, as mentioned above. Loosening the squeezing force to decongest the blood may result in an insufficient force for preventing shifting of the sensing section from the measuring object. As a result, the Requirement (2) is not met either.

### DISCLOSURE OF INVENTION

The present invention is made in view of the above problems, and it is an object of the present invention to provide a human-wearable biological signal measurement device which allows a user to adjust or maintain the device to/at a favorable state for a measurement without a need of much work and without imposing a burden.

In order to achieve the foregoing object, a biological signal measurement device of the present invention is a human-wearable biological signal measurement device, which device constantly or periodically measures a biological signal, including: a fitting section to be attached to a part of a human body; a sensing section, provided to the fitting section, which is to be closely attached to a specific part of the human body so as to sense the biological signal; a measurement processing section for performing a measurement of the biological signal by using the sensing section; a measurement environment judging section for judging a measuring environment based on an output of the sensing section; a measurement environment notifying section for notifying a user of a judgment result, according to a result given by the measurement environment judging section; and a measurement environment adjusting section for adjusting the measurement environment of the sensing section.

Further, in order to achieve the foregoing object a biological signal measurement device of the present invention is a human-wearable biological signal measurement device, which device constantly or periodically measures a biological signal, including: a fitting section to be attached to a part of a human body; a sensing section, provided to the fitting section, which is to be closely attached to a specific part of the human body so as to sense the biological signal; a measurement processing section for performing a measurement of the biological signal by using the sensing section; a measurement environment detecting section for detecting the measurement environment of the sensing section; a measurement environment judging section for judging the measurement environment based on an output of the measurement environment detecting section; a measurement environment notifying section for notifying a user of a judgment result, according to a result given by the measurement environment judging section; and a measurement environment adjusting section for adjusting the measurement environment of the sensing section.

Further, in order to achieve the foregoing object, a biological signal measurement device of the present invention is a human-wearable biological signal measurement device, which device constantly or periodically measures a biological signal, including: a fitting section to be attached to a part of a human body; a sensing section, provided to the fitting section, which is to be closely attached to a specific part of the human body so as to sense the biological signal; a measurement processing section for performing a measurement of the biological signal by using the sensing section; a measurement environment detecting section for detecting the measurement environment of the sensing section; a measurement environment judging section for judging the measurement environment based on an output of the measurement environment detecting section; and a measurement environment adjusting section for adjusting the measurement environment of the sensing section according to a result given by the measurement environment judging section.

Further, in order to achieve the foregoing object, a biological signal measurement method of the present invention is a biological signal measurement method to be worn by a human body, which method constantly or periodically measures a biological signal, comprising the steps of: (I) judging a measurement environment for sensing a biological signal through a close contact to a specific part of the human body; (II) giving notification to a user when the measurement environment is judged as to be unfavorable; and (III) executing a predetermined measurement of a biological signal when the measurement environment is judged as to be favorable.

Further, in order to achieve the foregoing object, a biological signal measurement method of the present invention is a biological signal measurement method to be worn by a human body, which method constantly or periodically measures a biological signal, comprising the steps of: (I) judging a measurement environment for sensing a biological signal through a close contact to a specific part of the human body; (II) adjusting the measurement environment when the measurement environment is judged as to be unfavorable; and (III) executing a predetermined measurement of a biological signal when the measurement environment is judged as to be favorable.

Additional objects, features, and strengths of the present invention will be made clear by the description below. Further, the advantages of the present invention will be evident from the following explanation in reference to the drawings.

### BRIEF DESCRIPTION OF DRAWINGS

Fig. 1 is a front view showing a human-wearable sensor of Embodiment 1 according to the present invention.
Fig. 2 is a plane view showing the human-wearable sensor of Embodiment 1 according to the present invention.
Fig. 3 is a perspective view showing the human-wearable sensor of Embodiment 1 according to the present invention.
Fig. 4 is a diagram showing fitting of the human-wearable sensor of Embodiment 1 according to the present invention.
Fig. 5 is a transparent view showing a fitting section and a measurement environment adjusting section of Embodiment 1 according to the present invention.
Fig. 6 is a cross-sectional view showing respective structures of the fitting section, the measurement environment adjusting section, and the measurement-condition determining section of Embodiment 1 according to the present invention.
Fig. 7 is a block diagram showing the human-wearable sensor of Embodiment 1 according to the present invention.
Fig. 8 is a flow chart of a measurement process of Embodiment 1 according to the present invention.
Fig. 9 is a flow chart of a measurement process of Embodiment 1 according to the present invention, in which process reliability is improved.
Fig. 10 is a flow chart showing how the measurement is periodically performed by the human-wearable sensor.
Fig. 11 is a plane view of a human-wearable sensor which notifies a measurement environment by means of audio output.
Fig. 12 is a plane view of a human-wearable sensor which notifies a measurement environment by means of audio output and liquid crystal display.
Fig. 13 is a cross-sectional view showing a human-wearable sensor of Embodiment 2 according to the present invention.
Fig. 14 is a block diagram showing the human-wearable sensor of Embodiment 2 according to the present invention
Fig. 15 is a flow chart of a measurement process of Embodiment 2 according to the present invention.
Fig. 16 is a cross-sectional view of a human-wearable sensor of Embodiment 3 according to the present invention.
Fig. 17 is a block diagram showing the human-wearable sensor of Embodiment 3 according to the present invention.
Fig. 18 is a flow chart of a measurement process of Embodiment 3 according to the present invention.
Fig. 19 is a cross-sectional view showing a human-wearable sensor of Embodiment 4 according to the present invention.
Fig. 20 is a block diagram showing the human-wearable sensor of Embodiment 4 according to the present invention.
Fig. 21 is a flow chart of a measurement process of Embodiment 4 according to the present invention.
Fig. 22 is a front view showing a human-wearable sensor of Embodiment 5 according to the present invention.
Fig. 23 is a diagram showing fitting of the human-wearable sensor of Embodiment 5 according to the present invention.
Fig. 24 is a front view showing a human-wearable sensor of Embodiment 6 according to the present invention.
Fig. 25 is a plane view showing the human-wearable sensor of Embodiment 6 according to the present invention.
Fig. 26 is a perspective view showing the human-wearable sensor of Embodiment 6 according to the present invention.
Fig. 27 is a diagram showing a fitting of the human-wearable sensor of Embodiment 6 according to the present invention.
Fig. 28 is a cross-sectional view showing the human-wearable sensor of Embodiment 6 according to the present invention.
Fig. 29 is a front view showing another human-wearable sensor of Embodiment 6 according to the present invention.
Fig. 30 is a diagram showing a fitting of the another human-wearable sensor of Embodiment 6 according to the present invention.
Fig. 31 is a front view showing a case where the human-wearable sensor of Embodiment 7 according to the present invention is worn in such a manner that the measurement environment adjusting section (rotation adjusting section) touches a back of a finger.
Fig. 32 is a front view showing a case where the human-wearable sensor of Embodiment 7 according to the present invention is worn in such a manner that the measurement environment adjusting section (rotation adjusting section) touches the finger cushion side of a finger.

### BEST MODE FOR CARRYING OUT THE INVENTION

### (Embodiment 1)

The following describes with reference to figures specific embodiments of the present invention. Note that members and functions that are the same in other embodiments are given the same symbols, and detailed explanations for these members and functions are not repeated.

As an example of a circular or hyperbolic biological signal measurement device worn on a part of a human body, the following Embodiment 1 of the present invention describes a circular or hyperbolic ring-type biological signal measurement device (hereinafter, simply referred to as human-wearable sensor) which is worn on a finger for measuring a pulse wave and/or oxygen saturation. The human-wearable sensor is a device to be worn on a user's finger substantially at all time, and suitably measures pulsebeat, blood pressure, and even blood oxygen saturation from a pulse wave, .

Through the above-described principal, the human-wearable sensor of the present embodiment measures a biological signal regarding a pulse wave or oxygen saturation, by casting a light ray from an LED (Light Emitting Diode) on an artery (proper palmar digital artery or the like) on a side of a finger cushion, and receiving, via a light receiver, the light ray reflected on a tissue such as a blood vessel in the finger. The human-wearable sensor enables adjustment of a suitable measurement environment and maintaining of the suitable measurement environment, or feedbacks adjustment conditions or measurement conditions, thereby realizing a highly reliable, highly accurate, and stable measurement.

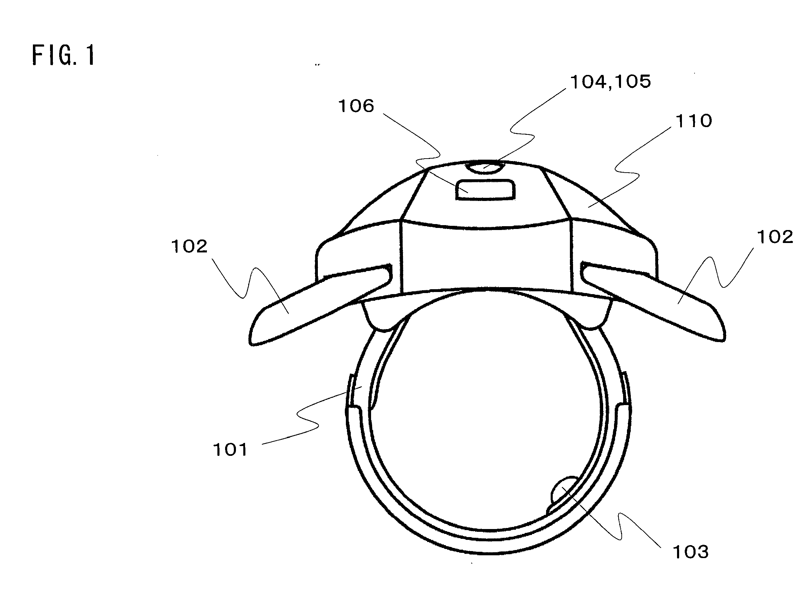

Fig. 1 to Fig. 4 shows a profile of the human-wearable sensor 100 of the present embodiment.

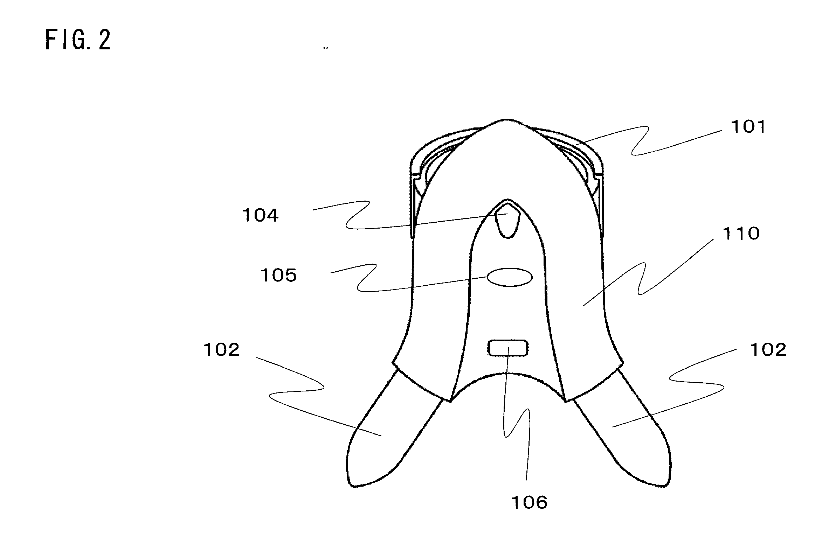

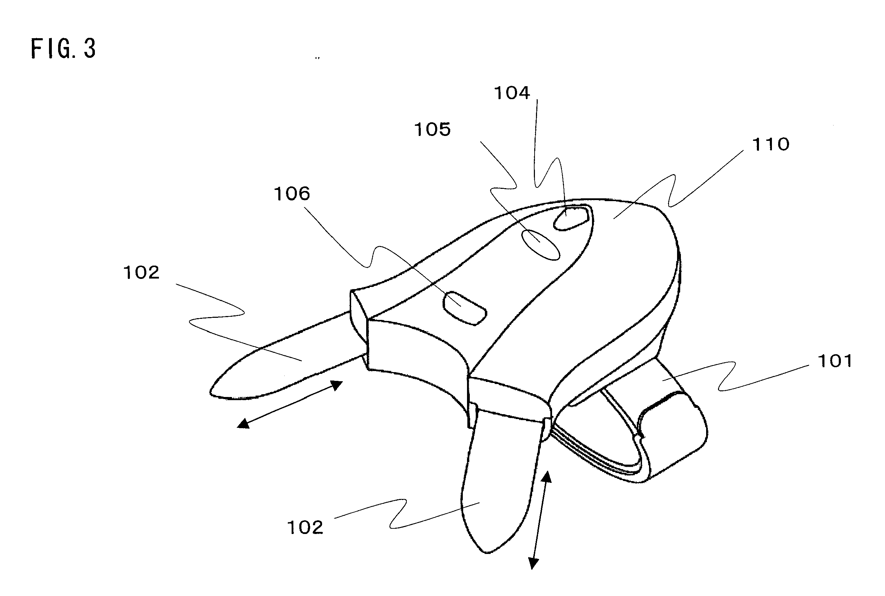

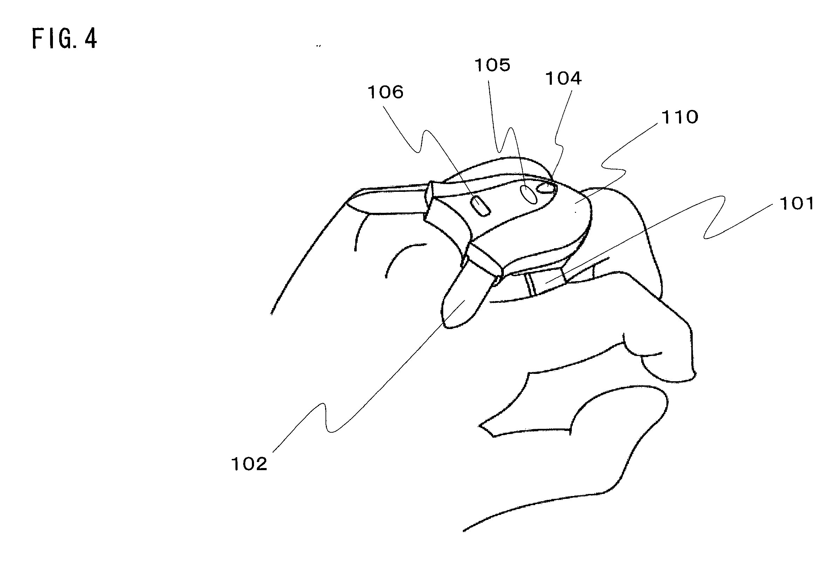

Fig. 1 is a front view of the human-wearable sensor 100, and Fig. 2 and Fig. 3 are respectively plane view and perspective view of the human-wearable sensor 100. Further, Fig. 4 shows the human-wearable sensor 100 worn on a finger.

The human-wearable sensor 100 of the present embodiment includes: a circular fitting section 101 into which a finger is inserted in such a manner that the fitting section 101 is positioned between a metacarpophalangeal joint and a proximal interphalangeal joint; measurement environment adjusting sections 102 for adjusting how the human-wearable sensor 100 is fit on the finger (hereinafter, fitting-state) by the fitting section 101; a sensing section 103 fixed on the fitting section 101, for optically sensing a predetermined signal from the human body, the sensing section 103 having a light emitting element and a light receiving element; and a main body 110. The main body 110 includes: a circuit board for realizing a measuring function or the like; a display section 104 functioning as a measurement environment notifying section for notifying a user of biological signal sensing conditions or sensing environment of the sensing section 103; an operation button switch 105 functions as an operating section of the main body; and a fitting-condition determining section 106 for use in releasing or fixing the measurement environment adjusting sections 102.

Next, a physical structure of each section is described.

To enable easier sensing of a signal from a blood vessel (measuring object), the fitting section 101 is formed in a circular shape: i.e., in shape and size such that the fitting section 101 fits on the base of finger (between the metacarpophalangeal joint and proximal interphalangeal joint of a finger). On the inner circumference of the fitting section 101, the sensing section 103 is provided. In the present embodiment, the measuring object of the human-wearable sensor 100 is an artery (proper palmar digital artery or the like) on the side of the finger cushion. Since the human-wearable sensor 100 is formed in such a manner as to fit on the joint of a finger as is the case of a normal ring, measurement can be performed without posing a problem in everyday life, unlike cases where a device is worn on a fingertip for example. Further, in a case of measurement at a fingertip which is a popular part of a body often targeted in conventional products, the measuring object is capillary vessels. On the other hand, the present invention performs measurement at the base of finger having an artery such as a proper palmar digital artery. Performing measurement with respect to a thick blood vessel such as the artery allows more accurate measurement.

The fitting section 101 is connected to the measurement environment adjusting sections 102. In the present embodiment, the fitting section 101 and the measurement environment adjusting sections 102 are made of a single belt-like member. The fitting section 101 is crossed within the main body 110. Both ends of the fitting section 101 project from the main body 110, forming projected portions which serve as the measurement environment adjusting sections 102.

Thus, by adjusting the length of each of the measurement environment adjusting sections 102 projecting from the main body 110, the tightness of wearing the human-wearable sensor 100 is adjusted according to the size of the user's finger. Further, the sensing section 103 is, for example, fixed on the fitting section 101 so as to move along with the fitting section 101. As such, the circumferential position of the sensing section 103 is adjusted by increasing the length of one of the two measurement environment adjusting sections 102, and reducing the length of the other.

Further, as shown in Fig. 4, the measurement environment adjusting sections 102 projecting from the main body 110 are designed to touch a part of the body other than the finger (e.g. back of the hand), thereby preventing rotation of the human-wearable sensor 100. In other words, when a force is applied to the human-wearable sensor 100, in a direction the human-wearable sensor 100 is able to rotate, either one of the two measurement environment adjusting sections 102 projected serves as a stopper by firmly touching the back of the hand.

With the structure, the user is able to freely adjust the squeezing force or the position of the sensing section 103. Thus, while the user is wearing the biological signal measurement device, the device is effectively kept from rotating without a need of over-squeezing the user's finger, and the accuracy of measurement is improved.

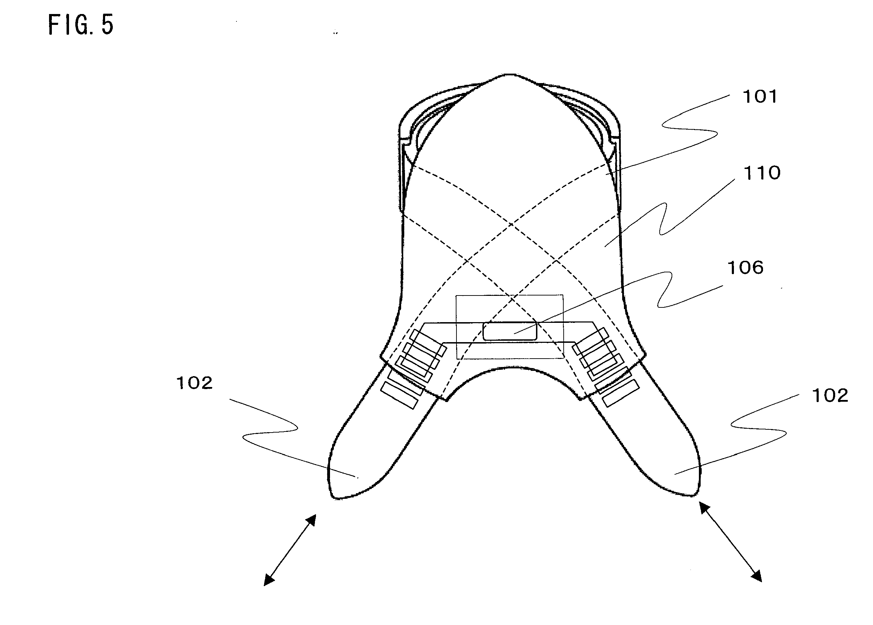

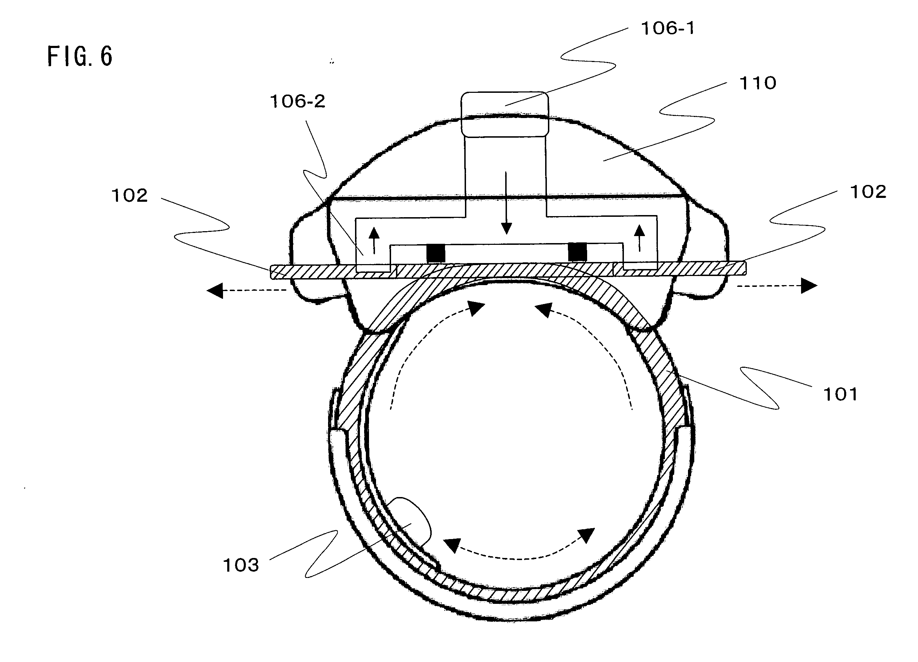

Fig. 5 is a plane view showing a perspective projection of the main body 110 to clarify the relation amongst: the fitting section 101; the measurement environment adjusting section 102; the main body 110; and the measurement-condition determining section 106. Further, Fig. 6 is a cross-sectional view of the human-wearable sensor 100. As mentioned above, the fitting section 101 and the measurement environment adjusting section 102 are formed by a single belt-like member. The fitting section 101 is crossed inside the main body 110, and the both ends of the fitting section 101 project from the main body 110 and form the measurement environment adjusting sections 102. Each of the belt-like shaped measurement environment adjusting sections 102 is provided with five grooves. The measurement-condition determining section 106 is made of an elastic member. A first portion 106-1 of the measurement-condition determining section 106 is partially exposed on the main body 110. Each of second portions 106-2 of the measurement-condition determining section 106 fixes the fitting section 101 and the measurement environment adjusting section 102 by being caught in one of the grooves of the measurement environment adjusting section 102. Note that each of the measurement environment adjusting sections 102 is provided with five grooves. In Fig. 5, both of the second portions 106-2 are caught in the middle grooves (third groove from an end) of the measurement environment adjusting sections 102.

As shown in Fig. 6, when the user pushes the first portion 106-1 of the measurement-condition determining section 106, the second portions 106-2 of the measurement-condition determining section 106 are lifted due to the leverage principal, and exit the respective grooves of the measurement environment adjusting sections 102. Note that the fulcrum of the leverage is shown by the black squares in the figure. For the sake of understanding, the respective directions of the members' movements are shown by the solid arrows in the figure. By holding and moving the measurement environment adjusting sections 102, it is possible to move the measurement environment adjusting sections 102 and the fitting section 101 in the directions shown by the dotted arrows in the figure. The sensing section 103 is fixed on the fitting section 101 so that it move with the fitting section 101. The user is able to freely adjust (i) the relative position of the sensing section 103 to the user's finger and (ii) the pressing force to the user's finger, by pressing the first portion 106-1 of the measurement-condition determining section 106.

Specifically, when the two measurement environment adjusting sections 102 projecting from the main body 110 are both pulled out by the same amount, the inner diameter of the fitting section 101 is reduced and the pressing force applied by the sensing section 103 to a part of the body is increased. By contrast, when the measurement environment adjusting sections 102 are pushed in by the same amount, the inner diameter of the fitting section 101 is increased, and the pressing force applied to the part of the body is reduced. Further, when one of the measurement environment adjusting sections 102 is shortened and another one of the measurement environment adjusting sections 102 is lengthened by the amount that one of the measurement environment adjusting sections 102 is shortened, the sensing section 103 fixed on the fitting section 101 moves in the circumferential direction of the fitting section 101.

Note that the structures of fitting section 101, the measurement environment adjusting section 102, the rotation prevention adjustment 105, and the measurement-condition determining section 106 shown in the present embodiment are mere examples, and these members are not limited to these. Further, the fitting section 101 and the measurement environment adjusting section 102 are physically formed in a single piece: i.e., made of a single belt. However, it is needless to say that the fitting section 101 and the measurement environment adjusting section 102 may be separately formed.

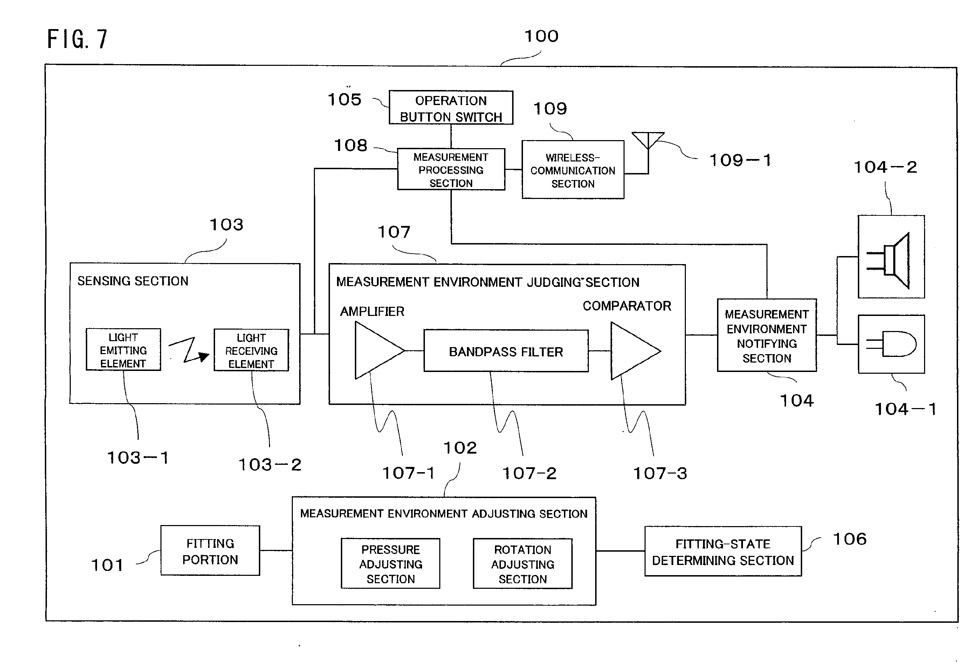

Fig. 7 is a block diagram of functional blocks of the human-wearable sensor 100 of the present embodiment. The human-wearable sensor 100 includes, as its functional members: the fitting section 101 which enables the human-wearable sensor 100 to be worn on the part of the human body; the measurement environment adjusting section 102 for adjusting an environment under which the sensing section performs measurement (hereinafter, measurement environment of the sensing section); the sensing section 103 for sensing a biological signal; the measurement environment notifying section 104 for notifying the user of the measurement environment of the sensing section; the operation button switch 105 for providing an input function with which the user controls each function of the human-wearable sensor 100; the fitting-state determining sections 106 for determining the fitting-state of the fitting section 101; a measurement environment judging section 107 for judging a measurement environment of the sensing section 103; a measurement processing section 108 for calculating predetermined biological information out from a signal obtained via the sensing section 103 (by driving the sensing section 103); and a wireless-communication section 109 for (i) transmitting to an external device the biological information having been calculated out by the measurement processing section, and for (ii) receiving an measurement instruction from an external device.

The mechanical members of the human-wearable sensor 100 are: the fitting section 101, the measurement environment adjusting section 102, and the fitting-state determining sections 106. The measurement environment adjusting section 102 has two functional members: i.e., a pressure adjustment section whose function is to adjust pressing of the sensing section 103 against a part of the body; and a position adjustment section for adjusting the relative position of the sensing section 103 to an artery in the direction of rotation. Note however that, in the present embodiment, these two functions are realized by a single member as described above. The fitting-state determining sections 106 allows changing of adjustment state or fitting state by the measurement environment adjusting section 102.

Next, the electric members of the human-wearable sensor 100 are: the sensing section 103; the measurement environment notifying section 104; the operation button switch 105; the measurement environment judging section 107; the measurement processing section 108; and the wireless-communication section 109. The sensing section 103 includes: a light emitting element 103-1 which is an LED; and a light receiving element 103-2 which is a PIN - PD (PIN - Photo Diode). The light emitting element 103-1 and the light receiving element 103-2 are closely located to each other, and are closely attached to the finger with a predetermined pressure. Light emitted from the light emitting element 103-1 is dispersed by and reflected on a tissue such as a blood vessel in the finger, received by the light receiving element 103-2, and is transmitted in a form of electric signal to the measurement environment judging section 107 and the measurement processing section 108. As mentioned above, the signal from the sensing section 103 is input to measurement environment judging section 107 for judgment of the measurement environment. This means that the sensing section also serves as a measurement environment detecting section for detecting the measurement environment.

The measurement environment notifying section 104 outputs as needed whether or not the environment for measurement by the sensing section 103 is favorable, which is judged by the measurement environment judging section 107. The result of the judgment is output by using a display section 104 -1 including a lamp (e.g. LED or the like) and/or a speaker 104-2 each connected to the measurement environment notifying section 104.

The operation button switch 105 provides the user with operation means via which the user is able to, for example, (i) stop the notification performed by the measurement environment notifying section 104 or (ii) turning on/off the power. Specifically, by pressing the operation button switch 105 and holding it down, the power is turned on or turned off. Pressing of the operation button switch 105 while a notification is made by the measurement environment notifying section 104 will stop the notification process.

The measurement environment judging section 107 includes an amplifier 107-1, a bandpass filter 107-2, and a comparator 107-3. In the present embodiment, the measurement environment is judged based on the accuracy of a signal sensed. The method of sensing is described below. It is assumed that a pulse wave is sensed.

As mentioned above, a hemoglobin in blood does not absorb very much of red color light while it is bond with oxygen, but absorbs red color light without oxygen. This characteristic is utilized as follows. When light from the light emitting element 107-1 is properly cast on a blood vessel, the light receiving element 103-2 receives a signal of light which varies according to pulsation of the blood vessel: i.e., expansion and constriction of the blood vessel. Accordingly, a signal with a waveform corresponding to the pulsation is obtained. On the contrary, when the light emitted from the light emitting element 103-1 is off the position of the blood vessel and light is not properly cast thereon, the light receiving element 103-2 is not able to receive signals that varies according to the pulsation. That is, when the sensing section 103 is properly worn on a position where measurement with respect to the blood vessel is possible, a signal having a shape of a waveform is obtained. Therefore, a signal of a large amplitude (difference between a highest point and a lowest point of a waveform of the signal) is obtained. However, when the sensing section is not properly worn, the amplitude is small. Thus, by looking at the amplitude, it is possible to know whether or not the sensing section 103 is properly worn.

Here, it is assumed that, when the amplitude is large, the accuracy is high and the measurement environment is favorable. Light is cast on the blood vessel from the light emitting element 103-1, and a signal received by the light receiving element 103-2 is measured. In order to use the signal and to simplify the judgment of the amplitude, the signal is amplified by the amplifier 107-1. The amplified signal is input to the bandpass filter 107-2 which only passes signals within a predetermined frequency band, and attenuates signals of other frequencies. Through this bandpass filter 107-2, the signal is shaped so that the amplitude of the signal is easily judged. Then, the comparator 107-3 judges whether or not the amplitude of the signal surpasses a predetermined criterion value. Here, to check if a pulse wave (measuring object) is properly sensed, the bandpass filter 107-2 extracts the main component of the pulse wave, and removes noise stemming from disturbance light, vibration, or the like. As such, in the present embodiment, the bandpass filter 107-2 passes frequency components of 0.1Hz to 20Hz, and restrains other frequency components. When the value of the amplitude of the signal does not surpass the criterion value, it is judged that the signal is not successfully received. On the contrary, when the value of the amplitude is more than the criterion value, it is judged that the signal is successfully received. This method of judging the measurement environment based on the accuracy of the signal is mere an example. The method is not limited to this method, and other methods are also possible.

The measurement processing section 108 drives the sensing section 103 to calculate predetermined biological information from a quantity of light received by the light receiving element 103-2 of the sensing section 103, which quantity is converted into an electric signal. For example, the biological information calculated out is a blood oxygen saturation, healthiness of the user, or the like. A specific method of calculating the biological information is a known technology, and an explanation for the specific method is omitted here. Further, the role of the measurement processing section 108 is not only to execute the measurement via the sensing section 103 and calculation of the biological information, but also to perform other controls in general, including execution of an operation entered via the operation button switch 105, communication with an external device by means of the later-described wireless-communication section 109. The measurement processing section 108 performs these operations based on a program stored in storage means built therein.

The wireless-communication section 109 transmits the biological information measured by the measurement processing section 108 to an external device, by means of wireless communication such as IEEE802.11 or Bluetooth^{®}, via an antenna 109-1. Meanwhile, the wireless-communication section 109 receives control information from an external device. For example, the control information is an instruction to execute measurement, or setting information. The information is received via the antenna 109-1, and is transmitted to the measurement section 108.

With reference to the figures, the following describes a flow of an operation performed by the human-wearable sensor 100.

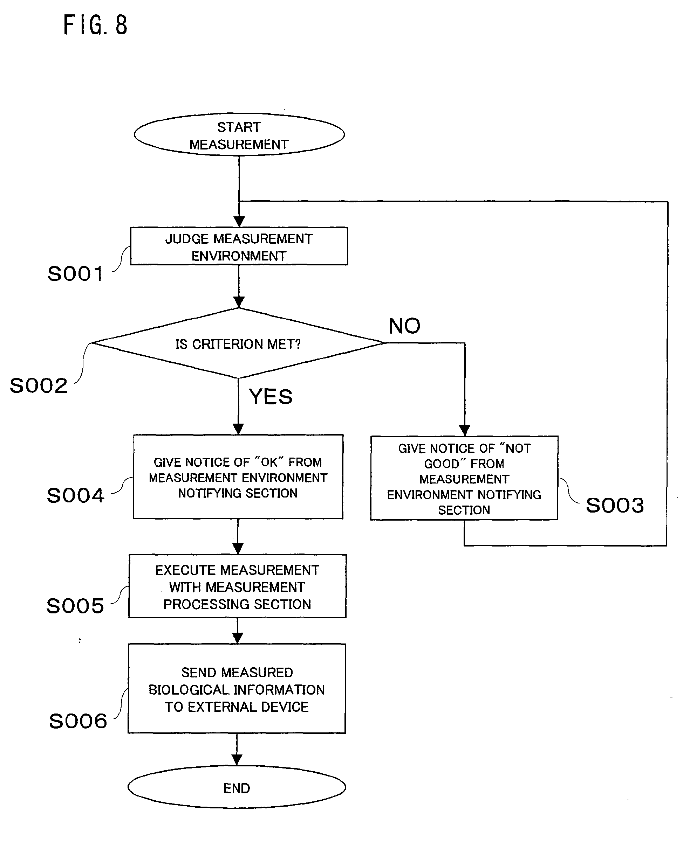

Fig. 8 shows a basic flow of the measurement process. Note that an explanation for a process performed prior to the start of the measurement (such as a process of turning the power on) is omitted here.

As shown in Fig. 8, when the measurement processing section 108 starts measurement, the measurement environment judging section 107 judges the measurement environment in the step (hereinafter, simply referred to as S) 001. The process of judgment is as mentioned hereinabove.

Next, in S002, if the measurement environment judging section 107 judges that the measurement environment is unfavorable, the operation goes to S003. If the measurement environment judging section 107 judges that the measurement environment is favorable, the operation goes to S004.

In S003, the measurement environment judging section 107 drives the measurement environment notifying section 104 to notify the user that the measurement environment is unfavorable. Specifically, the measurement environment judging section 107 causes the display section 104-1 having red and green LED lamps to blinks the red LED lamp. Further, the measurement environment judging section 107 causes the speaker 104-2 to make an alarm sound. Then, the operation returns to S001. Note that, to judge the measurement environment in S001, it is necessary to detect several heart beats. Therefore, while the unfavorable measurement environment is maintained, the red LED lamp blinks and the alarm sound is made from the speaker 104-2, at an interval of two seconds. It is needless to say that, in a case where blinking and making the alarming sound are set to last two seconds, the notification is continuously performed.

In S004, the measurement environment notifying section 104 causes the green LED lamp of the display section 104-1 to blink a certain length of period. Then, in S005, the measurement processing section 108 drives the sensing section 103 to perform measurement according to the operation principal described above. Finally, in S106, the measurement processing section 108 transmits the biological information having been measured to an external device via the wireless-communication section 109. In this case, the biological information is transmitted to a mobile phone of the user of the present human-wearable sensor 100. Then, the measurement is completed.

If a favorable measurement environment is not ensured even by operating the measurement environment adjusting section 102 and if the notification performed by the measurement environment notifying section 104 therefore does not stop, it is possible to stop the notification by pressing the operation button switch 106.

The human-wearable sensor 100 operating as described above is always worn on the user's finger. Accordingly, when the sensing section S103 is under a favorable measurement environment, the measurement is completed without particularly disturbing the user. When the sensing section S 103 is under an unfavorable measurement environment, the measurement environment notifying section 104 notifies the user of the unfavorable measurement environment. Thus, the measurement is properly performed.

That is, the measurement environment is judged upon start of the measurement. Then, until a favorable measurement environment is achieved, the measurement environment notifying section 104 continuously or periodically notifies that the measurement conditions are unfavorable. Thus, by adjusting the measurement environment adjusting section 102 until the measurement environment notifying section 104 stops notifying the unfavorable measurement conditions, the user is able to achieve favorable measurement conditions for the human-wearable sensor 100. Here, by operating (locking) the fitting-state determining sections 106 when the favorable measurement conditions are achieved, the human-wearable sensor 100 is retained at the suitable position. Further, the measurement environment adjusting section 102 can not only adjust the pressure and position of the sensing section 103, but also prevent rotation of the human-wearable sensor 100. This more effectively maintains a suitable fitting-state of the human-wearable sensor 100. Meanwhile, while the human-wearable sensor 100 is worn, the measurement environment notifying section 104 performs notification, if the fitting-state of the human-wearable sensor 100 becomes unfavorable and an accurate measurement is not possible. This prevents inaccurate measurement under a unfavorable environment. In a case where the measurement is inaccurate, the measurement environment notifying section 104 notifies the unfavorable conditions until the conditions becomes suitable conditions. In that event, the user only needs to perform readjustment.

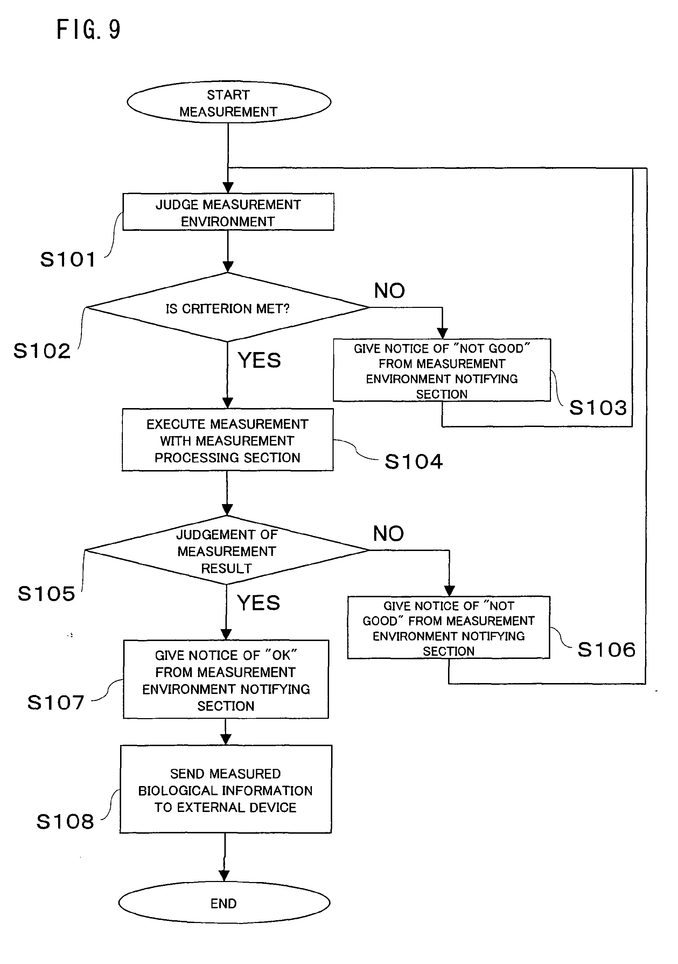

Fig. 9 shows a flow of the measurement process which enables more reliable measurement. In this example, the human-wearable sensor 100 is used as is the previous example. Note that an explanation for a process performed prior to the start of the measurement (such as a process of turning the power on) is omitted here.

As shown in Fig. 9. When the measurement processing section 108 starts measurement, the measurement environment judging section 107 judges the measurement environment in S101. The process of judgment is as described above.

Next, in S102, if the measurement environment judging section 107 judges that the measurement environment is unfavorable, the operation goes to S103. If the measurement environment judging section 107 judges that the measurement environment is favorable, the operation goes to S104.

In S103, the measurement environment judging section 107 drives the measurement environment notifying section 104 to notify the user that the measurement environment is unfavorable. Specifically, the measurement environment judging section 107 causes the display section 104-1 having red and green LED lamps to blink the red LED lamp. Further, the measurement environment judging section 107 causes the speaker 104-2 to intermittently make an alarm sound. Then, the operation returns to S001. The notification process can be stopped by pressing the operation button switch 106, as mentioned above.

The operation goes to S104 if the measurement environment is judged as to be favorable in S102, and the measurement processing section 108 drives the sensing section 103 to perform measurement according to the operation principal described above. Next, in S105, the measurement processing section 108 judges whether or not the measurement result is obtained through a proper measurement. This judgment is performed in addition to the judgment performed by the measurement environment judging section 107 on if the amplitude of a predetermined frequency component is not less than a predetermined amplitude. Through the judgment, it is possible to detect the following based on a waveform resulted from the measurement: a unfavorable measurement environment attributed to disturbance noise; and a measurement failure due to insufficient battery. The judgment also detects a measurement failure in spite of a suitable measurement environment during S101 which failure attributed to shifting of the position or disturbance noise occurred before or during the measurement. These are not detected by the measurement environment judging section 107.

If the measurement environment is judged as to be unfavorable in S104, the operation goes to S106. If the measurement environment is judged as to be favorable, the operation goes to S107.

In S106, the measurement environment judging section 107 drives the measurement environment notifying section 104 to notify the user that the measurement environment is unfavorable. Specifically, the measurement environment judging section 107 causes the display section 104-1 having red and green LED lamps to turn on the red LED lamp. Further, the measurement environment judging section 107 causes the speaker 104-2 to make a continuous alarm sound. Then, the operation returns to S001. The notification process can be stopped by pressing the operation button switch 106, as mentioned above.

The operation goes to S107 if the measurement environment is judged as to be favorable in S105, and the measurement environment notifying section 104 causes the green LED lamp of the display section 104-1 to blink a certain length of period. Then, in S108, the measurement processing section 108 transmits the biological information having been measured to an external device via the wireless-communication section 109. In this case, the biological information is transmitted to a mobile phone of the user of the present human-wearable sensor 100. Then, the measurement is completed.

As described, by performing, in addition to the judgment of the measurement environment (S101), the judgment (S 105) based on a signal resulted from the actual measurement, it is possible to prevent a measurement failure attributed to a cause (e.g., noise from disturbance light, insufficient battery, etc.) which is not detected by the measurement environment judging section 107. This enables further reliable process of sensing a biological signal. Further, unfavorable measurement conditions are notified to the user in different forms depending on whether (i) they are detected by the measurement environment judging section 107 or (ii) they are detected in an actual measurement and not detected by the measurement environment judging section 107. Thus, the user is able to suitably respond to each circumstance, knowing how the unfavorable measurement conditions are detected.

Here, in the above-described structure, the sensing section 103 is expected to pressed against a finger at a suitable pressure for measurement. However, depending on the purposes of use of the present human-wearable sensor 100, there may be cases where the measurement only needs to be performed periodically at a long interval, and the user therefore wants to have the sensing section 103 loosened during the ordinary time. In such cases, it is possible to periodically perform the measurement as shown in Fig. 8 or Fig. 9.

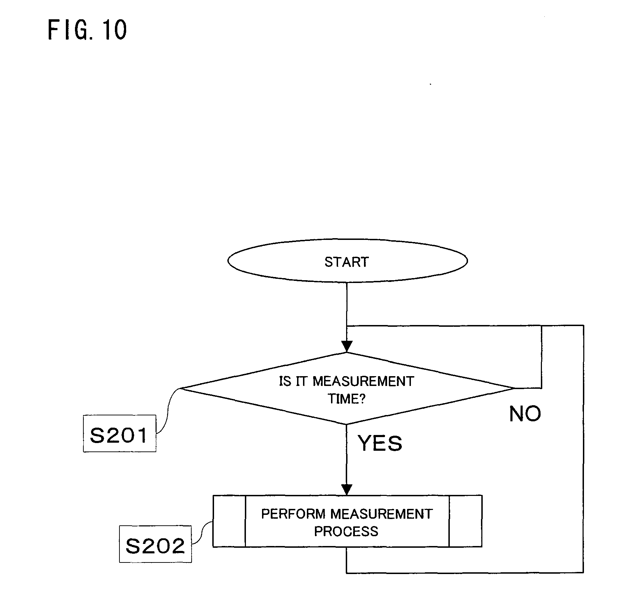

Fig. 10 shows a flow of an operation in which the human-wearable sensor 100 periodically performs measurement. In S201, the measurement processing section 108 uses a timer (not shown) to judge whether or not the time reached a predetermined measurement time which comes periodically once an hour. When the measurement time comes, the measurement shown in Fig. 8 or Fig. 9 is performed in S202.

In a case where the human-wearable sensor 100 is used for performing measurement in everyday life, the human-wearable sensor 100 is expected to be worn for a long period of time. This may afflict some users with a blood congestion caused by squeezing of the finger. Especially, a finger is a part of the body which largely varies in a single day. Depending on the time of performing the measurement and due to the body conditions, a finger may be swollen. If the finger is swollen, the user feels the finger is more tightly squeezed even with a squeezing pressure which does not make the user feel uncomfortable. Squeezing of the finger while the finger is swollen may even worsen the blood congestion. Even in this case, in the above-described measurement, the user is given a notice when a proper measurement likely to have failed. The user receives a notice when: (i) the band has been loosened, because the user was feeling uncomfortable or the user's finger became numb; or (ii) the measurement environment has changed after a long period of time due to variation in conditions of the finger such as swelling. Thus, it is possible to perform a suitable measurement. Of course, when the suitable measurement environment has been maintained, the measurement is performed without disturbing the user. When the suitable measurement environment is not maintained, the measurement environment adjusting section 102 re-sets the measurement conditions following the operation flow shown in Fig. 8 or Fig. 9.

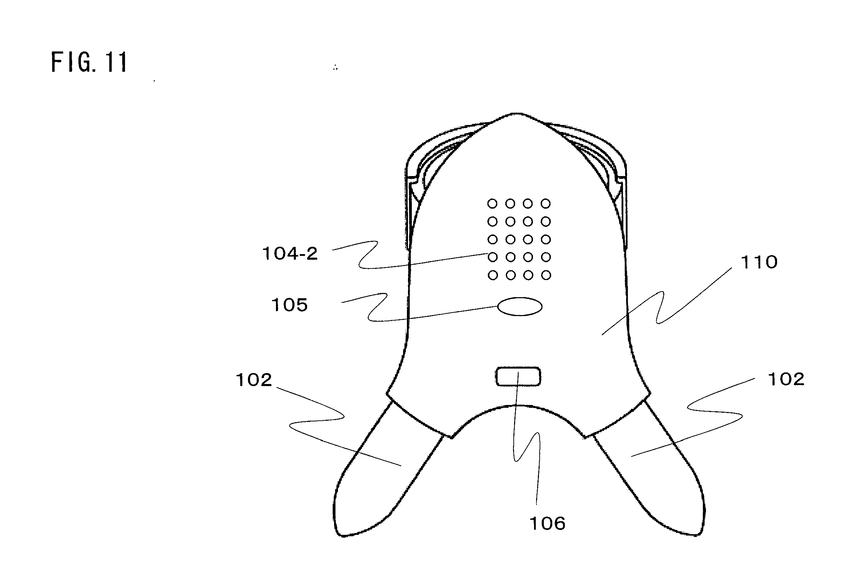

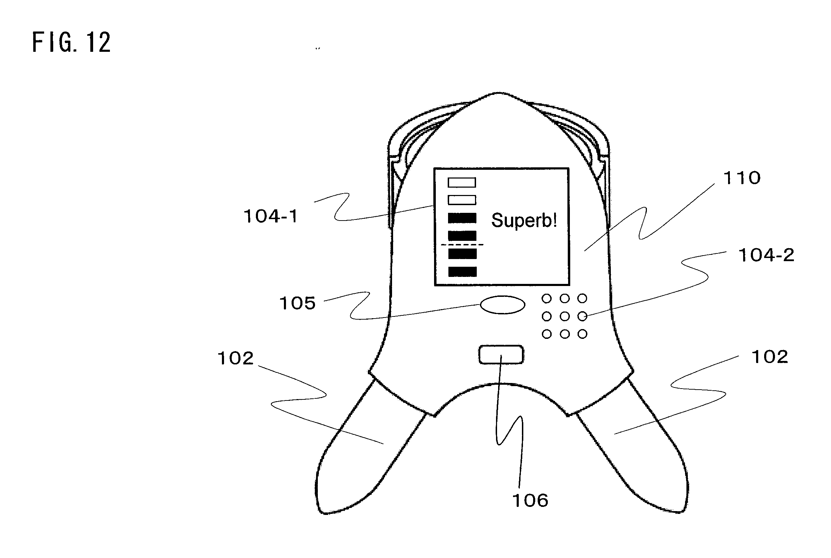

Each of Fig. 11 and Fig. 12 is a plane view showing an exemplary structure of a human-wearable sensor 100 having another measurement environment notifying section 104. Fig. 11 shows a case where a hole for the speaker 104-2 is perforated on the main body 110, so that the sound from the speaker 104-2 is efficiently output. Further, in the structure of Fig. 11, the display section 104-1 is omitted to cut the cost. Fig. 12 shows a case
where a liquid crystal display is used as the display section 104-1. In the case where the liquid crystal display 103 and speaker 103 are provided as shown in Fig. 12, for example, an unfavorable measurement environment can be displayed on the display section 104-1 in the form of text information reading "Not properly worn" or the like, while making an alarm sound from a speaker 102.

The above-described structures are mere examples of the measurement environment notifying section 104. It is needless to say that the measurement environment notifying section 104 is not limited to them, and that various structures of the measurement environment notifying section 104 are possible. For example, the measurement environment notifying section 104 may include a member that gives the user a notice by means of electric stimulation or vibration. It is possible to switch the output of the measurement environment notifying section 104 to such a member in an occasion where silence must be kept, for example.

Here, depending on the usage scenes (applications and services) of the biological signal sensed, there are believed to be various requirements in the accuracy of the method in which accuracy judgment is performed by the measurement environment judging section 107 based on a biological signal sensed by the sensing section 107. For example, in an application of the present invention to medical care aiming at treating a patient having an ailment or providing the patient with an aid in everyday life (e.g. preventing pulse beat or blood pressure from increasing due to a hard exercise), highly accurate biological information is required, and therefore a high accuracy is required in the judgment performed by the accuracy judging section 102. On the other hand, the present invention can be applied to a game or the like. For example, it is possible to change an effect (sound, vibration, or the like) or a story of the game according to the number of pulsebeat measured by the biological signal measurement device. In this case, the biological signal does not have to be so accurate, and rough sensing of biological signal would do. Therefore, the accuracy of judgment does not have to be so high either. As described, the accuracy is set according to the requirement of various usage scenes, and does not have to be fixed at a certain level for all the usage scenes. For this reason, it is preferable if the accuracy can be varied to a suitable level according to the usages scenes. Further, for example, it is possible to vary the accuracy by using control information obtained from a portable terminal such as a mobile phone, via the wireless-communication section 109.

### (Embodiment 2)

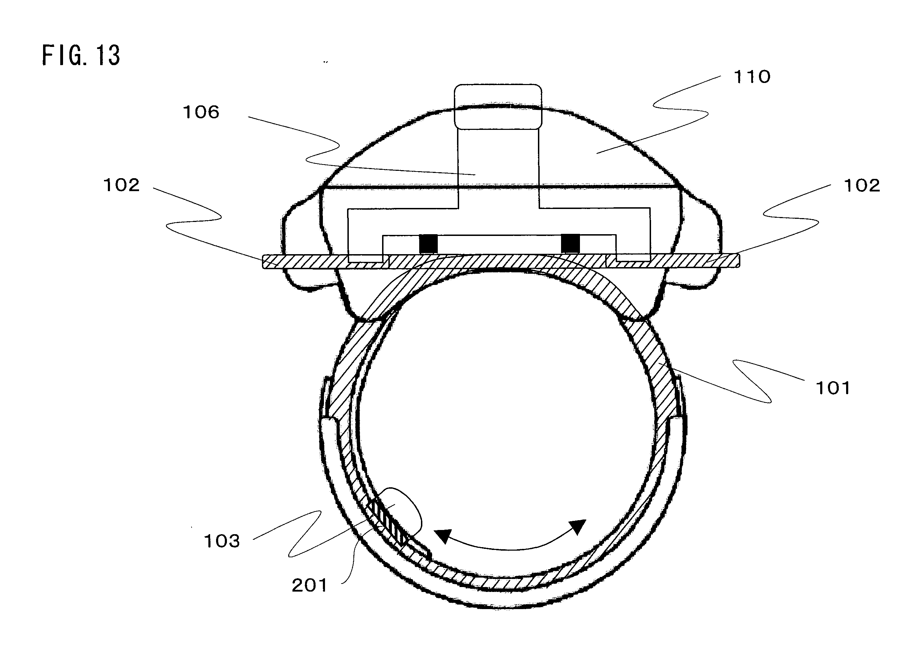

Fig. 13 shows a cross-sectional view of a human-wearable sensor 100 of Embodiment 2. As shown in Fig. 13, a pressure detecting section 201 is provided between a sensing section 103 and a fitting section 101, and measures a pressure applied to a part of a human body by the sensing section 103. As described, the pressure detecting section 201 functions as a measurement environment detecting section for detecting a measurement environment: i.e., a pressing force (pressure), applied by the sensing section 103. Note that, in the present embodiment, the pressure detecting section 201 is realized by a pressure sensor for directly measuring a pressure applied by the sensing section 103 in the vertical direction to the surface of the human body. There are various known technologies for the pressure sensor, and therefore no explanation is provided here regarding the structure of the pressure sensor.

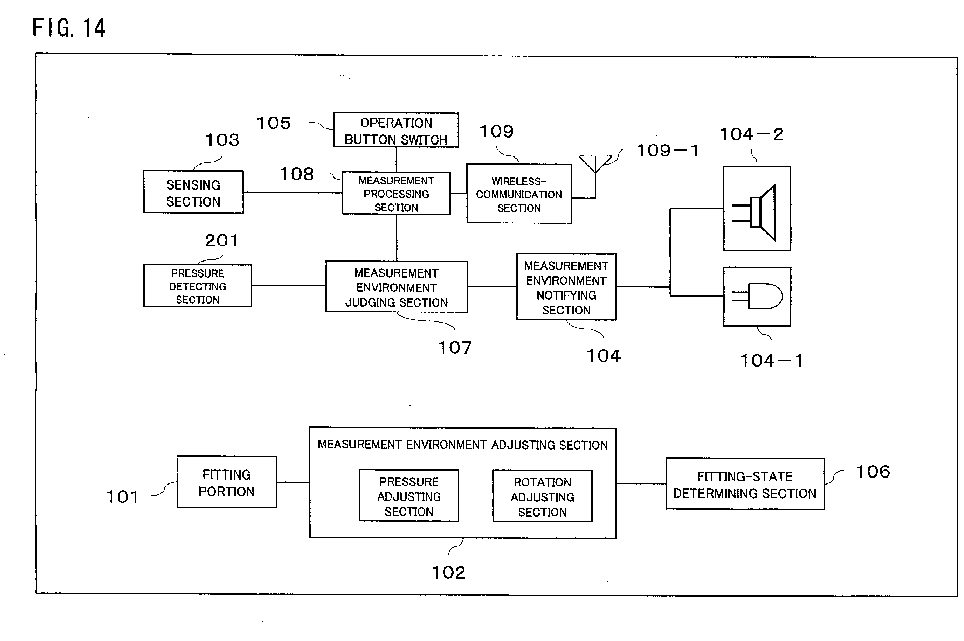

Fig. 14 is a block diagram showing functional structure of the human-wearable sensor 100 of the present embodiment. As shown in Fig. 14, the human-wearable sensor 100 includes, as its functional member, the pressure detecting section 201 in addition to: the fitting section 101; the measurement environment adjusting section 102; the sensing section 103; the measurement environment notifying section 104; the operation button switch 105; the fitting-state determining sections 106; the measurement environment judging section 107; the measurement processing section 108; and the wireless-communication section 109 each described with reference to Fig. 7. The pressure detecting section 201 is connected to the measurement environment judging section 107, and the measurement environment judging section 107 further judges an environment related to a pressure applied by the sensing section 103 to the part of the human body.

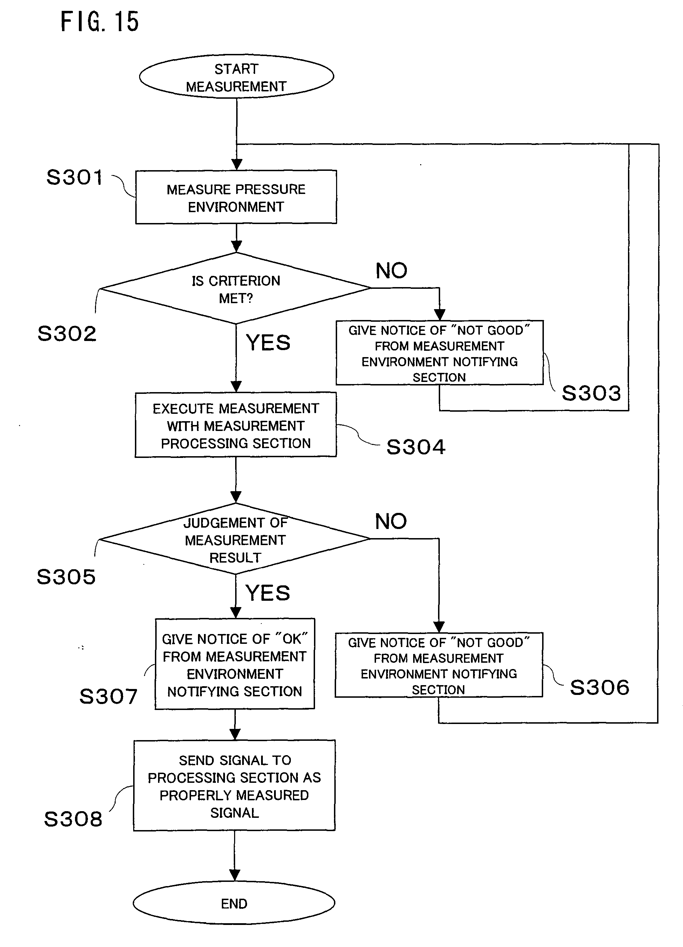

Fig. 15 shows an exemplary flow of a most-preferred measurement process using the human-wearable sensor 100 having the pressure detecting section 201 of the present embodiment. When measurement is started, the pressure detecting section 201 measures a pressure applied by the sensing section 103 to the part of the human body, in S301. Subsequently, in S302, the measurement environment judging section 107 judges whether or not the pressure is within a suitable range. Here, in this judgment, it is judged whether or not the pressure is within such a pressure range that suitable measurement is performed with respect to the proper palmar digital artery of the finger wearing the human-wearable sensor 100. If the pressure is not within the suitable range, the operation goes to S303, and the measurement environment notifying section 104 notifies the user that the pressure applied to the part of the body by the sensing section 103 is not within the suitable range. Here, the display section 104-1 of the present embodiment has a liquid crystal display section and a speaker section 102 as shown in Fig. 12. Thus, it is possible to make an alarm sound from the speaker 102, while indicating that the pressure is unfavorable, in the form of text information reading "Squeezing pressure of human-wearable sensor not suitable" or the like displayed on the display section 104 -1.

In S302, if the pressure is within the suitable range, the operation goes to S304, and the measurement processing section 108 performs a predetermined measurement of biological information. Subsequently, in S305, the measurement environment judging section 107 judges whether or not the measurement result is proper or not. In S305, the measurement processing section 108 performs a calculation process with respect to a waveform of the measurement result, thereby judging whether or not the measurement has been properly performed. This judgment is performed in addition to the judgment performed by the measurement environment judging section 107 of Embodiment 1 on if the amplitude of a predetermined frequency component is not less than a predetermined amplitude. Through the judgment, it is possible to detect the following based on a waveform resulted from the measurement: a unfavorable measurement environment attributed to disturbance noise; and a measurement failure due to insufficient battery. The judgment also detects a measurement failure in spite of a suitable measurement environment during S301 which failure attributed to shifting of the position or disturbance noise occurred before or during the measurement. These are not detected by the measurement environment judging section 107.

In S305, if the measurement result is not judged as to be normal, the operation goes to S306. In S306, the measurement environment notifying section 104 notifies the user that the measurement was not properly performed even though the pressure applied to the part of the body by the sensing section 103 was within the suitable range. Here, there the environment for optically sensing biological information from the proper palmar digital artery includes two main factors: i.e., the pressing force (pressure) applied to the body by the sensing section 103; and the relative position of the sensing section 103 to the blood vessel. Accordingly, in this process of notification, an alarm sound is made from the speaker 102 while text information reading "Please confirm the position of the human-wearable sensor" or the like is displayed on the display section 104-1.

In S305, if the measurement result is judged as to be appropriate, the operation goes to S307 and then to S308. Then, the measurement process is completed. The processes of S307 and S308 are the same as those of S107 and S108 mentioned above.

As described, the human-wearable sensor 100 of the present embodiment includes the pressure detecting section 201 for detecting the pressing force (pressure) applied to the part of the body by the sensing section 103. Thus, when a measurement environment is unfavorable, it is possible to specifically notify what caused the worsening of the measurement environment. That is, if the measurement environment notifying section 104 notifies that the pressure is unfavorable, the user simply needs to change the pressure by operating the fitting environment adjusting section 102. On the other hand, if the measurement environment notifying section 104 notifies that the position is unfavorable, the user simply needs to change the position by operating the fitting environment adjusting section 102. Thus, comparing to the human-wearable sensor 100 of Embodiment 1, the human-wearable sensor 100 of the present embodiment is able to give a user more suggestions regarding what to do next.

Note that the flow of Fig. 8 or Fig. 9 can be adopted to the human-wearable sensor 100 of the present embodiment shown in Fig. 14. That is, in the process of judging the measurement environment in S001 of Fig. 8 or S101 of Fig. 9, it is possible to have the pressure detecting section 201 judge the pressure condition. Alternatively, the measurement processing section 108 may further perform a test measurement in S001 or S101. Then, the environment judging section 107 may perform a calculation process with respect to data obtained through the measurement of the sensing section 103, so as to judge if the amplitude of a predetermined frequency component is not less than a criterion value. Then, judgment may be made by deriving a logical product of the result and the pressure condition detected by the pressure detecting section 201 in S002 or S102.

### (Embodiment 3)

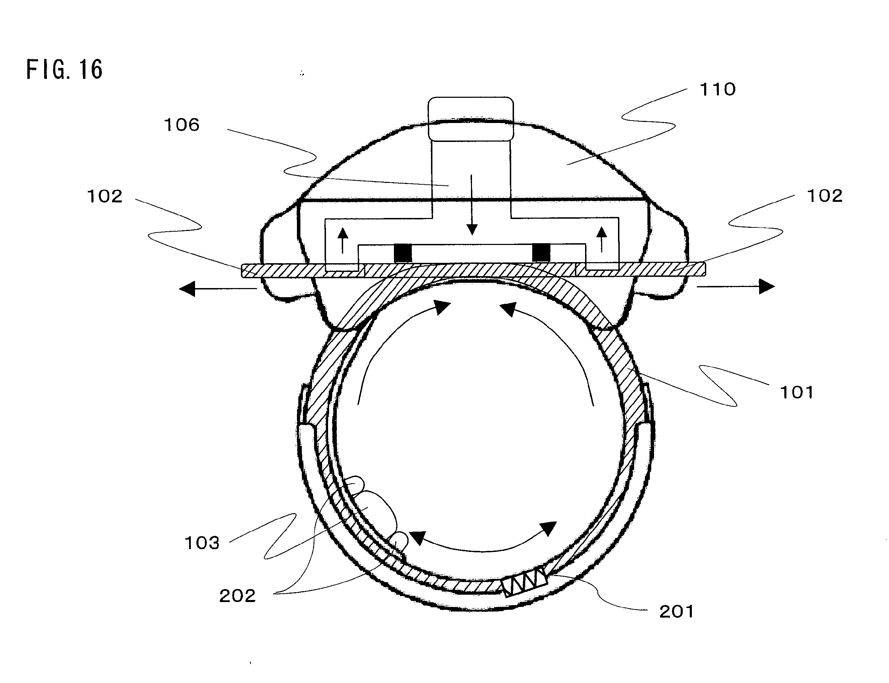

Fig. 16 is a cross-sectional view showing a human-wearable sensor 100 of Embodiment 3. As shown in Fig. 16, both ends of a pressure detecting section 201 are connected to the fitting section 101. In the present embodiment, the pressure detecting section 201 measures a tensile force generated by the fitting section 101, so as to indirectly measures a pressure applied to a part of a human body by the sensing section 103. There are various known technologies for a tensile force sensor, and therefore no explanation is provided here regarding the structure of the tensile force sensor. Here, if the fitting section 101 has a sufficient flexibility, a pressing force at the sensing section 103 and the tensile force detected by the tensile force sensor are linearly correlated to each other. Thus, by measuring the tensile force generated by the fitting section 101, the pressure applied to the part of the human body by the sensing section 103 is indirectly measured. Of course, the pressure detecting section may be arranged and structured as mentioned in Embodiment 2.

Next, on both sides of a the sensing section 103, position detecting sections 202 are provided closely to the sensing section 103. The position detecting sections 202 are for detecting a relative position of the sensing section 103 to a proper palmar digital artery (measuring object). Specifically, the position detecting sections 202 are two microphones, each of which detects a sound of blood stream flowing in the proper palmar digital artery (hereinafter, artery sound). Then, from a ratio of the artery sounds respectively detected by the both microphones, the relative position of the sensing section 103 to the proper palmar digital artery (measuring object) are detected. More specifically, the position detecting section 202 (two microphones) are arranged symmetrically in relation to the sensing section 103. The artery sounds detected by the two microphones are compared, and if the respective volumes of the artery sounds differ from each other by a predetermined amount, it is judged that the proper palmar digital artery is located in a position shifted from the sensing section 103 towards one of the microphones having detected an artery sound of the larger volume. On the other hand, if the difference between the artery sounds is not more than the predetermined amount, it is judged that the proper palmar digital artery is located in a suitable position in relation to the sensing section 103. As described, the position detecting section 201 functions as a measurement environment detecting section for detecting a measurement environment regarding the relative position of the sensing section 103 to a part of human body.

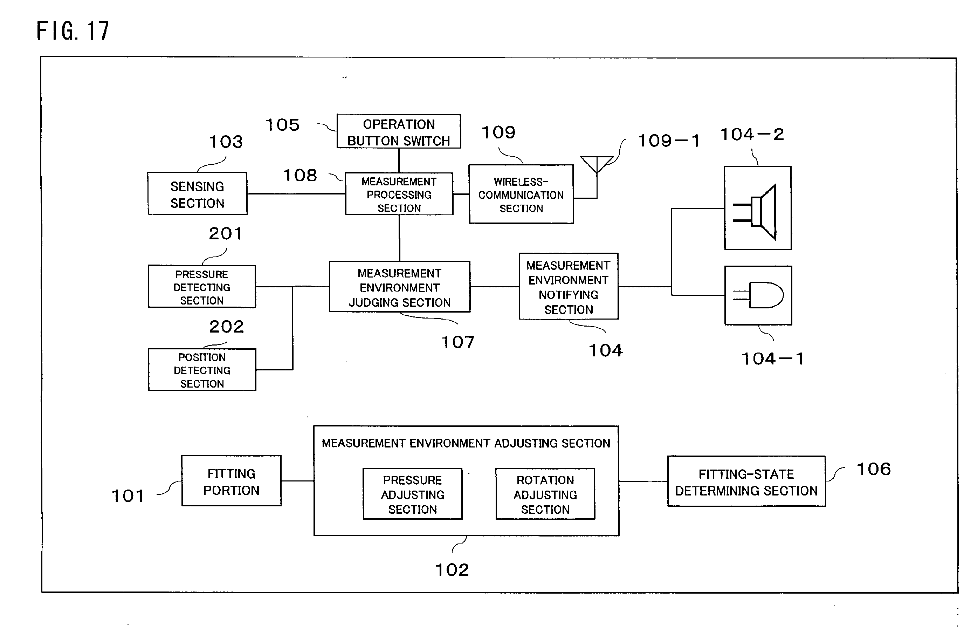

Fig. 17 is a block diagram showing a functional structure of the human-wearable sensor 100 of the present embodiment. As shown in Fig. 17, the human-wearable sensor 100 includes, as its functional member, the pressure detecting section 201 and the position detecting section 202, in addition to: the fitting section 101; the measurement environment adjusting section 102; the sensing section 103; the measurement environment notifying section 104; the operation button switch 105; the fitting-state determining sections 106; the measurement environment judging section 107; the measurement processing section 108; and the wireless-communication section 109 each described with reference to Fig. 5. The position detecting section 202 is connected to the measurement environment judging section 107, and the measurement environment judging section 107 further judges an environment related to a position of the sensing section 103 on the part of the human body, which position is detected by the position detecting section 202.

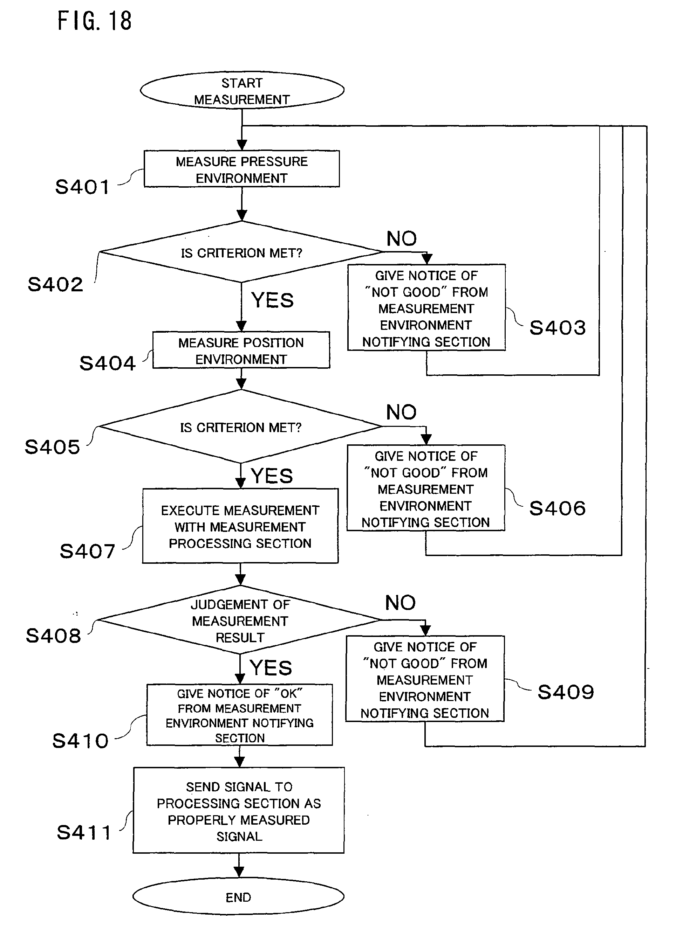

Here, the present embodiment allows further detailed environment judgment based on three pieces of environment information. An exemplary operation of this case is explained with reference to Fig. 18.

Fig. 18 shows an exemplary flow of a most-preferred measurement process using the human-wearable sensor 100 of the present embodiment. As shown in Fig. 18, processes from S401 to S403 are respectively the same as those of S301 to S303.

If the pressure is judged as to be within a suitable range in S402, the operation goes to S404. In S404, the measurement environment judging section 107 compares the artery sounds respectively detected by the position detecting sections 202 (two microphones), so as to measure the position of the sensing section 103 in relation to the proper palmar digital artery.

Subsequently, in S405, the measurement environment judging section 107 judges whether or not the position measured in S404 is a suitable position. If the position is judged as to be not suitable. The operation goes to S406, and the measurement environment notifying section 104 notifies that the position of the sensing section 103 is shifted in relation to the proper palmar digital artery (measuring object). Here, the display section 104-1 of the present embodiment has a liquid crystal display section and a speaker section 102 as shown in Fig. 12. Thus, it is possible to make an alarm sound from the speaker 102, while indicating that the position is unfavorable in the form of text information reading "Position of human-wearable sensor not suitable. Please rotate it towards the arrow." or the like displayed on the display section 104 -1. The operation then returns to S401.

In S405, if the position judged in S404 is a suitable position, the operation goes to S407, and the measurement processing section 108 performs a predetermined measurement process of biological information. Subsequently, in S408, the measurement environment judging section 107 judges whether or not the measurement result is appropriate. This judgment is the same as S305.

In S408, if the measurement result is not judged as to be normal, the operation goes to S410. In S410, the measurement environment notifying section 104 notifies that the measurement was not properly performed even though the pressure applied to the part of the body by the sensing section 103 was within the suitable range. In this case, none of the main causes (pressing force, and position) has contributed. Therefore, text information reading "Measurement Failed" or the like is displayed on the display section 104 -1.

In S408, if the measurement result is judged as to be appropriate, the operation goes to S410 and to S411. Then, the measurement process is completed. The processes of S410 and S411 are the same as those of S107 and S108 mentioned above.

As described, the human-wearable sensor 100 of the present embodiment includes the position detecting section 202 for detecting relative position of the sensing section 103 to a measuring object (in this case, proper palmar digital artery), in addition to the pressure detecting section 201 for detecting the pressing force (pressure) applied to the part of the human body by the sensing section 103. Thus, when the measurement environment is unfavorable, it is possible to more specifically notify what caused the worsening of the measurement environment. In other words, the reliability of notification made by the measurement environment notifying section 104 regarding unfavorable pressure and unfavorable position is improved. Regarding a unfavorable position, the sound volumes of sounds respectively detected by the two microphones 202 are compared to judge in which way the position is shifted. Then, detailed notification is made even in regard to which direction the position should be corrected. Thus, comparing to the human-wearable sensor 100 of Embodiment 2, the human-wearable sensor 100 of the present embodiment is able to give a user more suggestions regarding what to do next, including what to do with the fitting environment adjusting section 102.

Note that the flow of Fig. 8 or Fig. 9 can be adopted to the human-wearable sensor 100 shown in Fig. 17, too. That is, in the process of judging the measurement environment in S001 of Fig. 8 or S101 of Fig. 9, it is possible to judge the pressure condition and positional condition respectively by the pressure detecting section 201 and the position detecting section 202, based on a logical product. Here, a logical product of "1" means "favorable, and a logical product of "0" means unfavorable. Alternatively, the measurement processing section 108 may perform a test measurement in S001 or S101. Then, the environment judging section 107 may perform a calculation process with respect to data obtained through the measurement of the sensing section 103, so as to judge if the amplitude of a predetermined frequency component is not less than a criterion value. Then, judgment may be made by deriving a logical product of the result, the pressure condition detected by the pressure detecting section 201, and the positional condition detected by the position detecting section 202 in S002 or S102.

### (Embodiment 4)

Each of Embodiments 1 to 3 deals with a human-wearable sensor 100 which judges whether or not the measurement conditions are favorable, and notifies a user of the judgment result. However, a human-wearable sensor 100 of Embodiment 4 includes a fitting environment adjusting section 102 which automatically adjusts a measurement environment, according to the measurement environment having been judged by the measurement environment judging section 107.

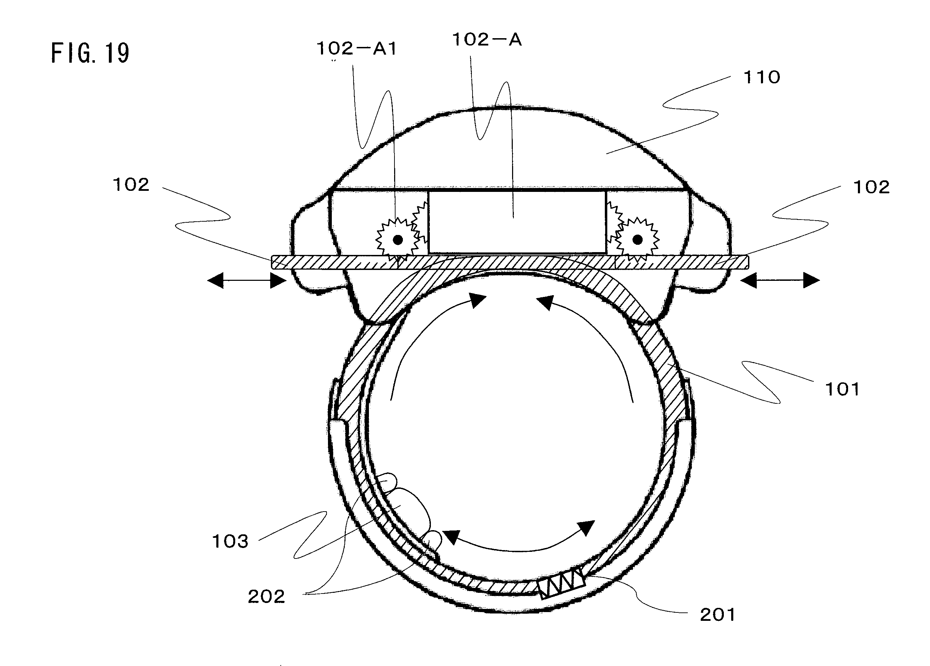

Fig. 19 is a cross-sectional view of the human-wearable sensor 100 of the present embodiment. The basic structure of the human-wearable sensor 100 of the present embodiment is as shown in Fig. 16. The measurement environment adjusting section 102 further includes a fitting adjustment drive section 102-A in addition to the members of the measurement environment adjusting section 102 shown in Fig. 16. The fitting adjustment drive section 102-A includes a small motor. This motor drives a gear 102-Al linked thereto so as to adjust the position of the sensing section 103, or the pressing force applied to a part of a human body by the sensing section 103.

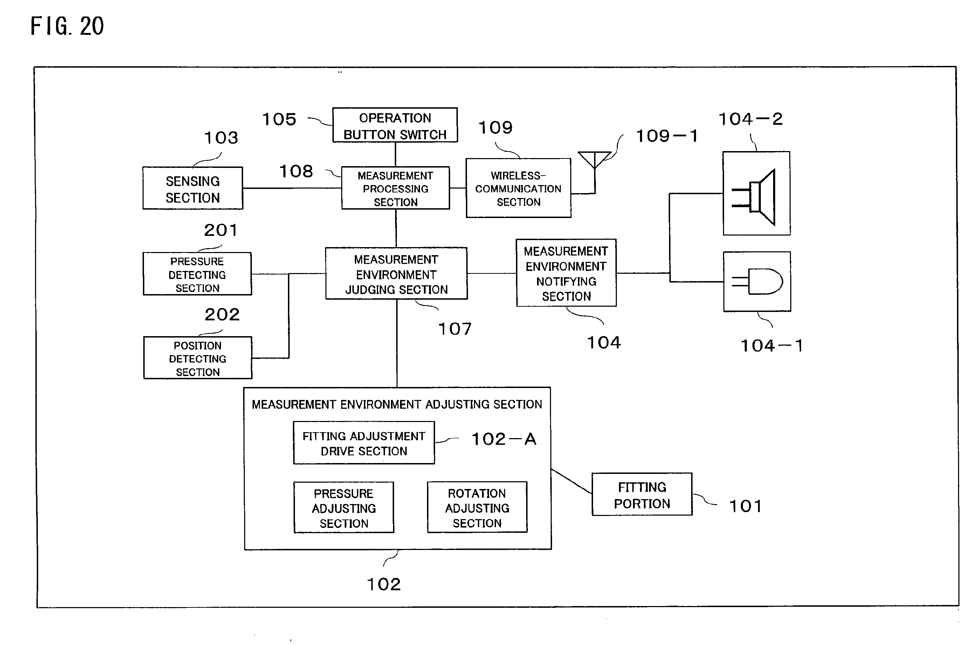

Fig. 20 is a block diagram showing a functional structure of the human-wearable sensor 100 of the present embodiment. The human-wearable sensor 100 includes, as functional members, the fitting adjustment drive section 102-A, in addition to: the fitting section 101; the measurement environment adjusting section 102; the sensing section 103; the measurement environment notifying section 104; the operation button switch 105; the measurement environment judging section 107; the measurement processing section 108; the wireless-communication section 109; the pressure detecting section 201; and the position detecting section 202 each described with reference to Fig. 17. The fitting adjustment drive section 102-A is connected to the measurement environment judging section 107, and the measurement environment judging section 107 obtains adjustment information for a measurement environment, which information is used in driving the measurement environment adjusting section.

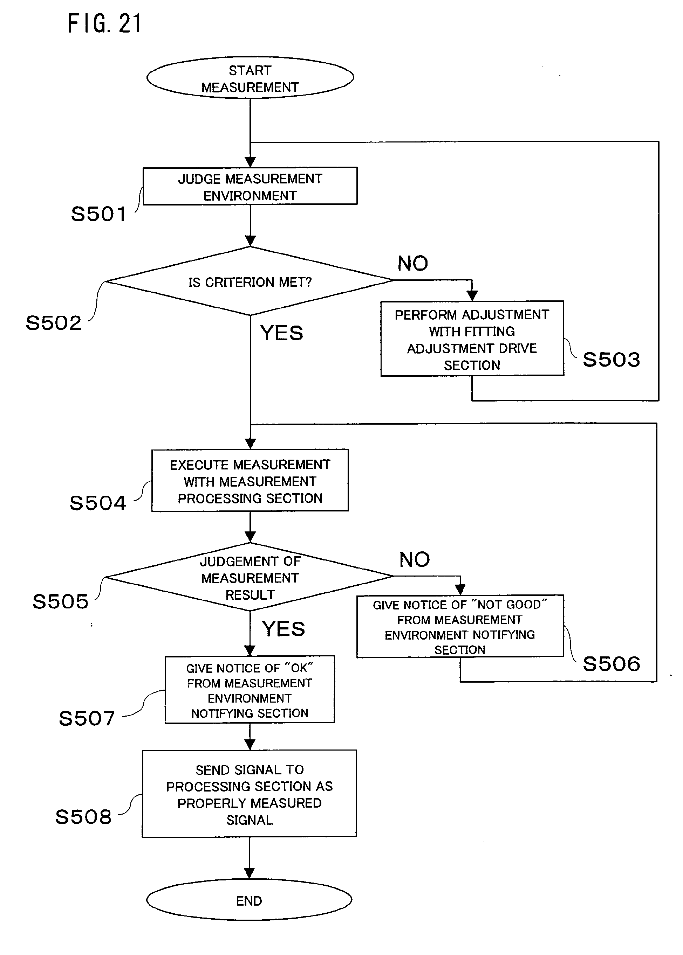

Fig. 21 shows an example of a most-preferred measurement operation flow using the human-wearable sensor 100 of the present embodiment. As shown in Fig. 21, when measurement is started, a measurement environment is first judged in S501. Specifically, a pressure environment and a position environment of the sensing section 103 are judged through the same process performed in S401 and S404. Subsequently, in S502, the measurement environment judging section 107 judges the measurement environment. If the measurement environment is judged as to be unfavorable, the operation goes to S503. In S503, the fitting adjustment drive section 102-A drives the measurement environment adjusting section 102. As described, in S501 through S503, the measurement environment is automatically adjusted, based on information output from the pressure detecting section 201 and the position detecting section 202, until the pressure environment and position environment of the sensing section 103 are suitable for the measurement.

In S502, if the measurement environment judging section 107 judges that the measurement environment is favorable, the operation goes to S504. Then, in S504, the measurement processing section 108 performs a predetermined measurement process with respect to biological information. Subsequently, in S505, the measurement environment judging section 107 judges whether or not the measurement result is appropriate.

In S505, if the measurement result is not judged as to be appropriate, the operation goes to S506. In S506, the measurement environment notifying section 104 notifies that the measurement was not properly performed even though the pressure applied to the part of the human body by the sensing section 103 and the position of the sensing section 103 are automatically adjusted. In this case, none of the main causes (pressing force, and position) has contributed. Therefore, text information reading "Measurement Failed" or the like is displayed on the display section 104 -1.

In S505, if the measurement result is judged as to be appropriate, the operation goes to S507 and to S508. Then, the measurement process is completed. In S507, a green LED lamp 104-1 blinks for a certain length of period. Then, in S 508, the measurement processing section 108 transmits the biological information obtained through the measurement to an external device via the wireless-communication section 109, and then the measurement process is completed. In this case, the external device is a mobile phone of the user of the present human-wearable sensor 100. Here, if the display section includes a liquid crystal display function as is 104-1 shown in Fig. 12, it is possible to notify the summary of the measurement result at the same time of notifying the succeeding of the measurement.

As described, with the human-wearable sensor 100 of the present embodiment, a measurement environment is automatically adjusted by the fitting adjustment drive section 102-A, based on information about a pressing force applied by the sensing section 103 and relative position of the sensing section 103 to the measuring object which information is obtained by the pressure detecting section 201 and the position detecting section 202.

As described in the present embodiment, if the human-wearable sensor 100 having the fitting adjustment drive section 102-A periodically performs measurement as is shown in Fig. 10, the measurement environment adjusting section 102 can be driven so that the fitting section 101 is loosened when the measurement is not performed, and that a suitable measurement environment is ensured only at the time of the measurement. The loosening of the fitting section 101 when the measurement is not performed will prevent compression or blood congestion caused by squeezing the user's finger, and relieve the unpleasant feeling of wearing the human-wearable sensor 100. Here, when conditions are adjusted by the measurement environment adjusting section 102 in the measurement performed for the first time, the adjusted conditions may be stored. Then, in the measurement performed after the first measurement, it is possible to restore the adjusted conditions by driving the measurement environment adjusting section 102, before the adjustment is started. This allows the adjustment to be completed in a short time in the measurement performed after the first measurement. Thus, it is possible to save the battery, and reduce the work of the user.

Note that the exemplary flow shown in Fig. 21 is made possible simply by adding the fitting adjustment drive section 102-A to the structure of Fig. 14 which is described in Embodiment 2. Since position and pressure are two main factors that disturb the measurement environment, it is very likely that the position is unfavorable when the pressure is judged as to be suitable. Thus, in the loop from S501 to S503 in Fig. 21, the pressure environment is first adjusted based on the output of the pressure detecting section 201. Then, in the same loop, the fitting adjustment drive section 102-A is operated to change the position while keeping the pressure constant, until a predetermined amplitude of a signal of a predetermined frequency is obtained from the sensing section 103.

Alternatively, when the position detecting section 202 is provided instead of the pressure detecting section 201, it is very likely that the pressure is unfavorable if the position detecting section 202 judges that the position is suitable. Thus, the flow of Fig. 21 can be performed by adjusting the pressure after the adjustment of the position.

Further, the exemplary flow shown in Fig. 21 can be also adopted even when the pressure detecting section and the position detecting section described in Embodiment 1 are not provided. That is, the sensing section is used also as the environment detecting section. In S501, the amplitude of a predetermined frequency component obtained from the sensing section is judged. Next, in S502, if an amplitude to be used as a criterion is not obtained, the following is performed in S503. The fitting adjustment drive section is driven to vary the relative position of the sensing section to the measuring object, while keeping a constant pressure of the sensing section to the human body, so as to obtained the largest amplitude. If the criterion in S502 is not met even in the loop from S501 to S503, the pressing force (pressure) applied by the sensing section to the human body is varied by the fitting adjustment drive section in S503, while keeping the sensing section 103 in a position where largest amplitude is obtained. Then, the pressure is kept being varied by the fitting adjustment drive section while keeping the relative position of the sensing section to the human body constant, in the loop from S501 to S503. In this way, the exemplary flow of Fig. 21 can be adopted to the other structure. Of course, it is possible to reverse the sequence: find the pressure at which the largest amplitude is obtained, while keeping the position constant; and then find the position where the largest amplitude is obtained, while keeping the pressure constant.

### (Embodiment 5)

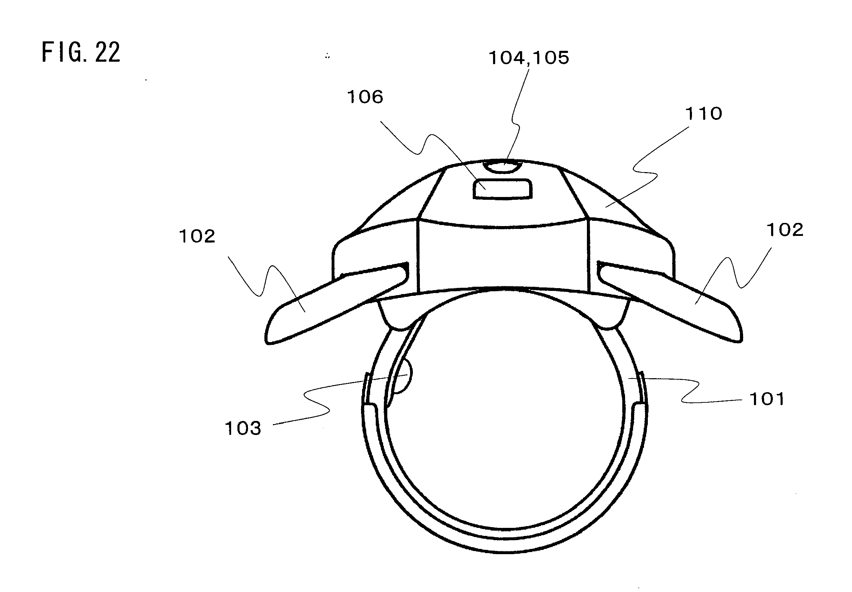

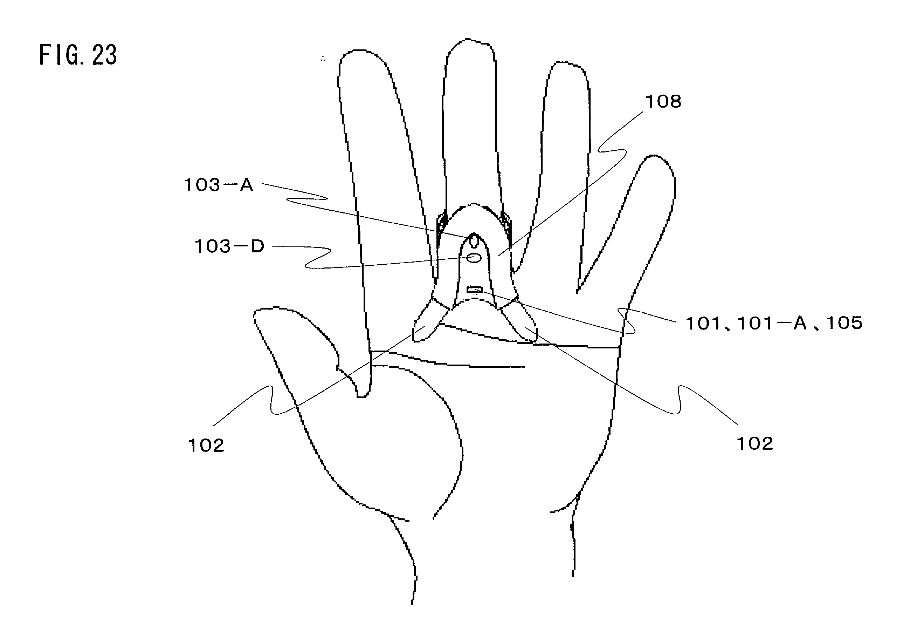

Each of the foregoing embodiments deal with a human-wearable sensor 100 which is worn on a finger in such a manner that the measurement environment adjusting section 102 is located on the backhand side of the finger. However, the human-wearable sensor 100 of the present embodiment is such that the measurement environment adjusting section 102 is located on the palm of the hand when wearing the human-wearable sensor 100. Fig. 22 is a front view of the human-wearable sensor 100 of the present embodiment. Fig. 23 is an outline view of the human-wearable sensor 100 being worn. Since a measuring object (blood vessel) is an artery (proper palmar digital artery) on the side of a finger cushion, a sensing section 103 is arranged more to the side of the main body 110 than the sensing section 103 of Fig. 1, so that the sensing section 107 touches the finger cushion side to be subjected to the measurement.

For example, when hands are free (e.g. during jogging), the main body 110 and the measurement environment adjusting section 102 can be located either on the backhand side or palm side, depending on the accuracy needed, and no particular problem will rise. However, for example, in a case of exercising with a bike exercising machine in a fitness club where hands are gripping the handles, the main body 110 and the measurement environment adjusting section 102 are preferably on the backhand side. Here, the sensing section 103 may be such that its position can be rotated by 180°. In other words, by enabling the position of the sensing section 103 in relation to the measuring object to be arranged according to the position of the measurement environment adjusting section 102 in various scenes, the human-wearable sensor 100 can be used in different scenes, and different forms of wearing the human-wearable sensor 100 according to the accuracy required are possible.

### (Embodiment 6)

Each of Embodiments 1 to 5 deals with a human-wearable sensor 100 which adjusts both the position of the sensing section 103 and a pressing force of the sensing section 103 to a part of a body. However, the human-wearable sensor 100 of the present embodiment described below includes a measurement environment adjusting section 102 of a different structure.

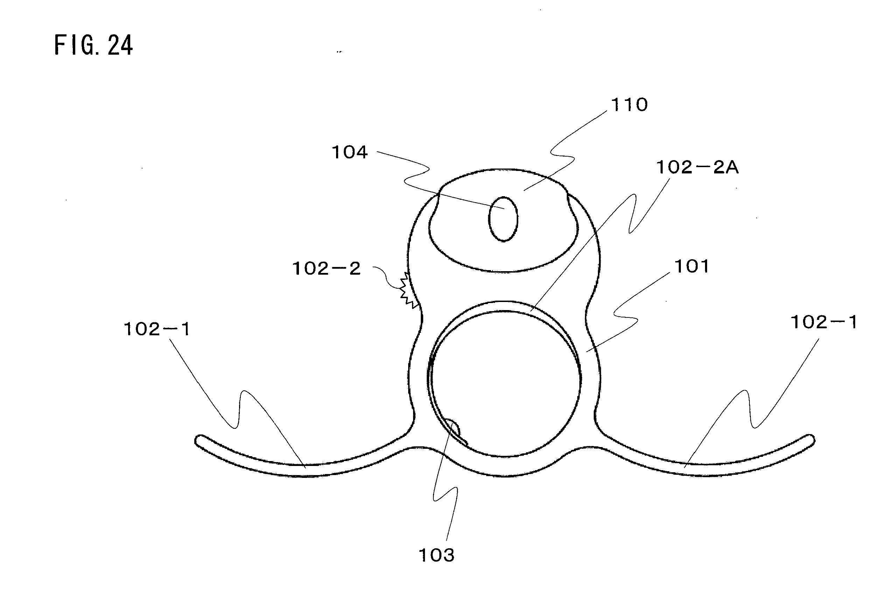

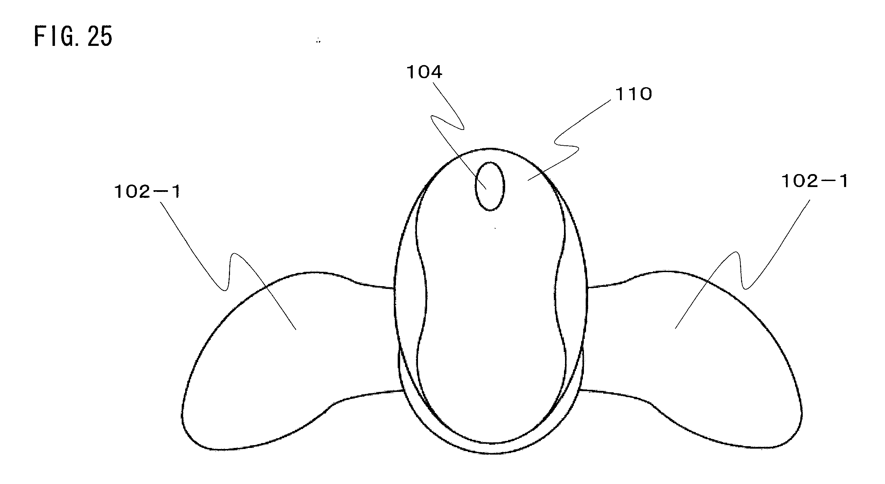

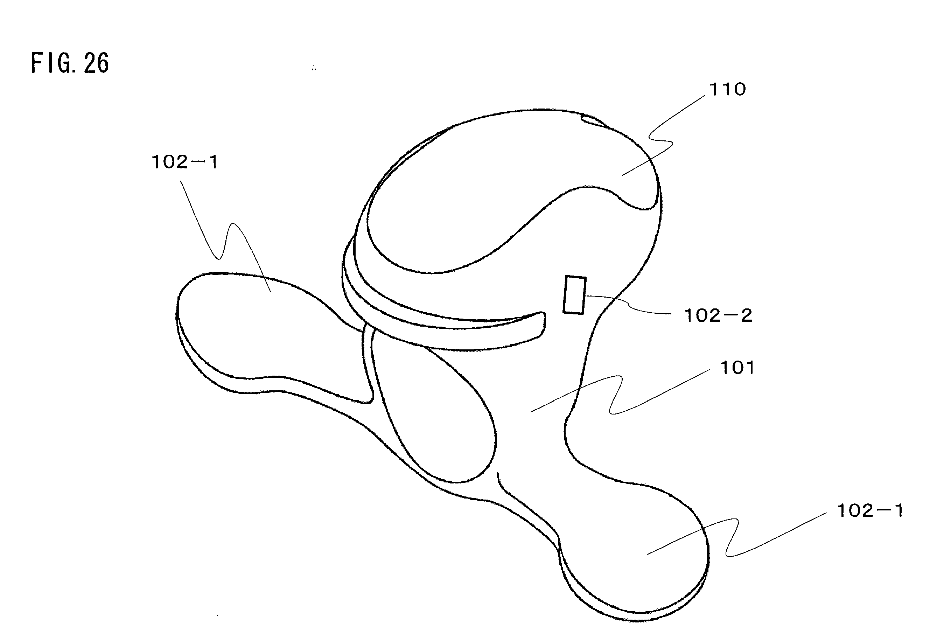

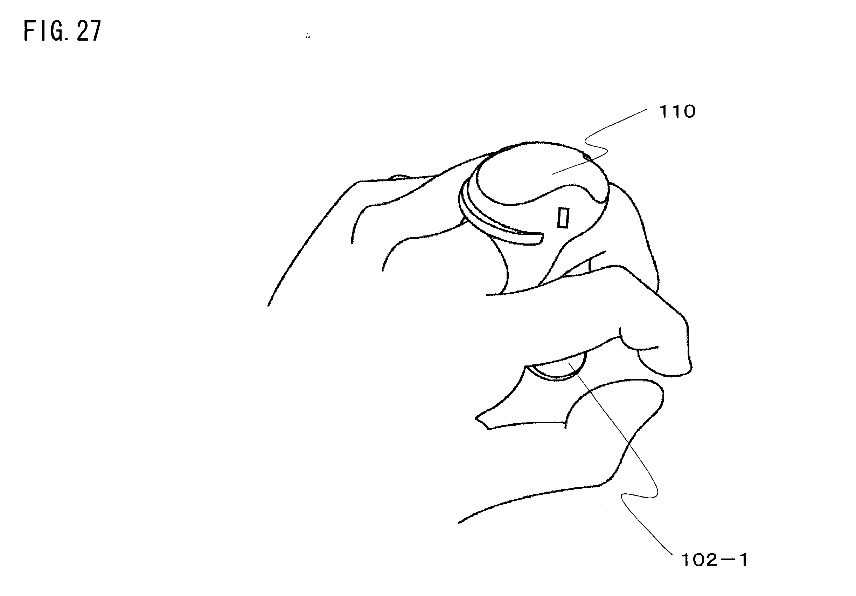

Fig. 24 is a front view showing the human-wearable sensor 100 of the present embodiment. Figs. 25 and 26 are respectively a plane view, a perspective view of the human-wearable sensor 100 of the present embodiment. Fig. 27 is a diagram showing the human-wearable sensor 100 of the present embodiment being worn. A measurement environment adjusting section (rotation adjusting section) 102-1 and a measurement environment adjusting section (pressure adjustment section) 102-2 have following functions for adjusting a measurement environment of a sensing section 103. The measurement environment adjusting section (rotation adjusting section) 102-1 prevents rotation by touching a finger cushion of an adjacent finger, and adjust the position of the detector 103 in the rotation direction. The measurement environment adjusting section (pressure adjustment section) 102-2 adjusts a pressing force applied to a part of the body by the sensing section 103 on a fitting section, by having a part 102-2A sticking out towards inner side of the fitting section 101, reducing the inner diameter of the circular shaped fitting section 101.

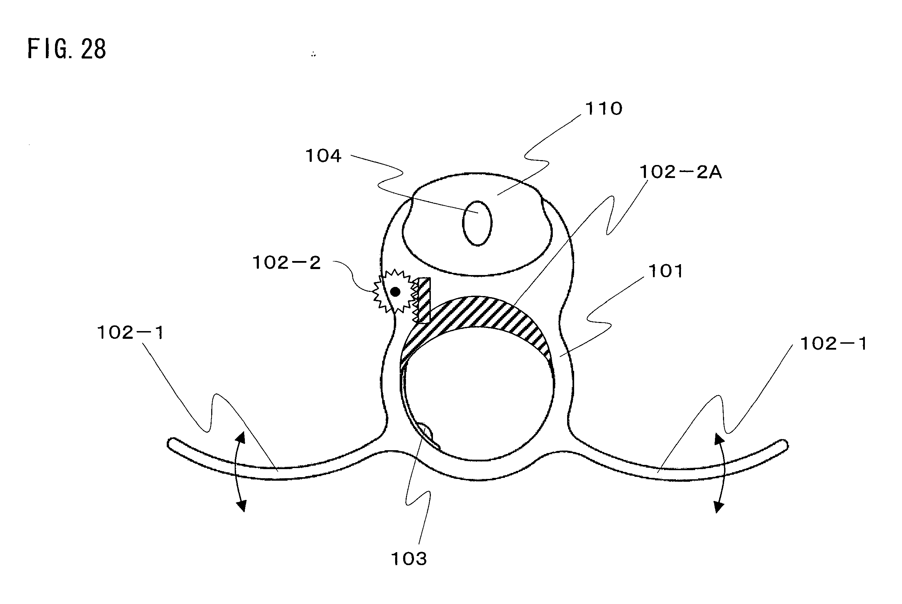

Fig. 28 is a cross-sectional view of the human-wearable sensor of the present embodiment. The measurement environment adjusting section (rotation adjusting section) 102-1 is made of an elastic member capable of maintaining its shape. For example, such a member is made of wire which can be deformed freely and which is capable of maintaining the deformation. This measurement environment adjusting section 102-1 can be deformed in the direction shown by the arrow in the figure. By adjusting an amount of the deformation, the measurement environment adjusting section 102-1 prevents rotation, and allows setting of the relative position of the sensing section 103 to a measuring object. The measurement environment adjusting section (pressure adjustment section) 102-2 includes a gear which is partially exposed on the main body 110. By operating the exposed part of the gear, the amount of the part 102-2A sticking out towards inside of the fitting section 101 is adjusted. By adjusting the amount of sticking out, a pressing force of the sensing section 103 to a part of the body can be adjusted.

As illustrated in Fig. 25 to Fig. 27, the measurement environment adjusting section (rotation adjusting section) 102-1 of the present embodiment has wings on both side, supposing that the reference line is a direction of a finger to be inserted into the fitting section 101. Here, it is supposed that a middle finger is to be subjected to a measurement, the both wings of the measurement environment adjusting section 102-1 are positioned so as to respectively touch the index finger and the third finger (hereinafter, supporting finger). Further, to allow the measurement environment adjusting section (rotation adjusting section) 102-1 to touch large portions of the supporting fingers and allow the user to stably hold the human-wearable sensor 100, the measurement environment adjusting section 102-1 is formed in a wing-like shape or a paddle-like shape so as to cover substantially all the finger cushions of the supporting fingers. As described, even a motion of the user causes an application of a force to the human-wearable sensor 100, the wing-like rotation adjusting sections touch the fingers on both sides of the finger subjected to the measurement, thereby preventing the rotation. Thus, further accurate measurement can be performed.

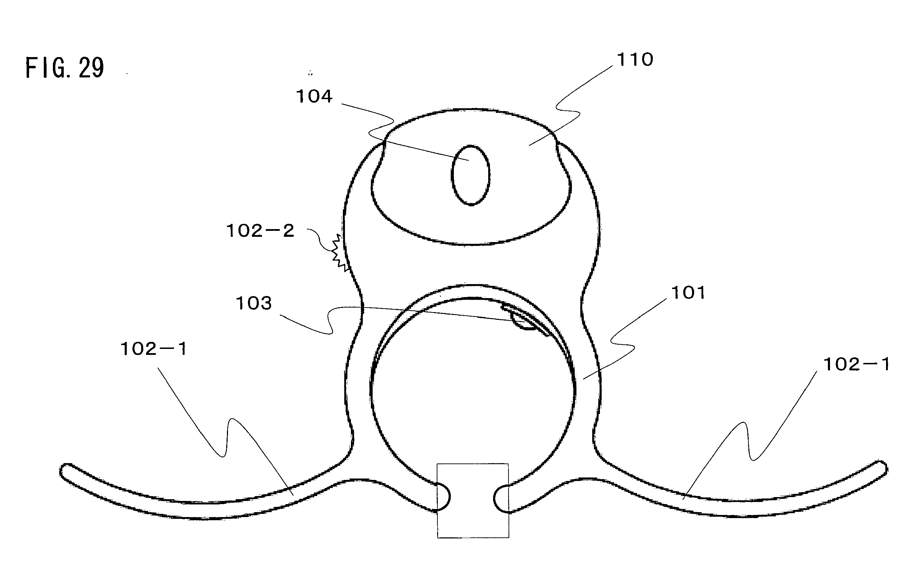

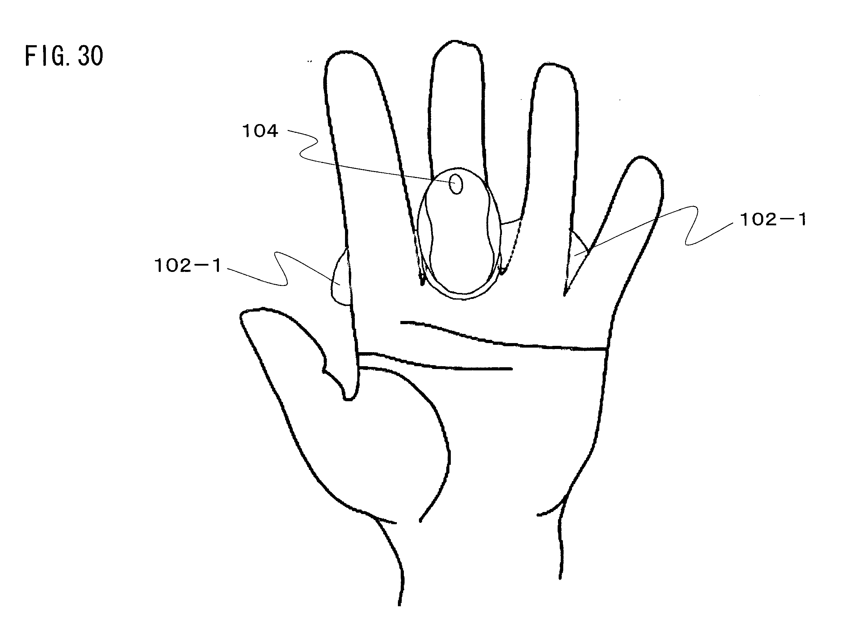

Further, Fig. 29 is a front view showing another exemplary structure of the present embodiment. The measurement environment adjusting section (rotation adjusting section) 102-1 is arranged so as to touch the back of the supporting fingers. Fig. 30 shows this measurement environment adjusting section 102-1 when it is being worn. Note that, in Fig. 29, the fitting section 101 is formed in a hyperbolic shape, instead of a circular shape. As shown in the figure, the fitting section may be formed in a hyperbolic shape.

### (Embodiment 7)

Embodiment 6 described a human-wearable sensor 100 whose measurement environment adjusting section (rotation adjusting section) 102-1 touches a finger cushion (Fig. 24 to Fig. 28), and a human-wearable sensor 100 whose measurement environment adjusting section (rotation adjusting section) 102-1 touches the backhand side of a finger (Fig. 29, Fig. 30). In these examples, the sensing section 103 is arranged so as to touch the measuring object (finger cushion). Therefore, different shapes have been adopted.

The present embodiment describes a measurement environment adjusting section (rotation adjusting section) 102-1 which is made of a further elastic material so that the measurement environment adjusting section 102-1 can be used in either cases, as long as it is possible to flip over an arch-shaped rotation preventing section 204 which touches a finger cushion or the back of a finger depending on the type of use.

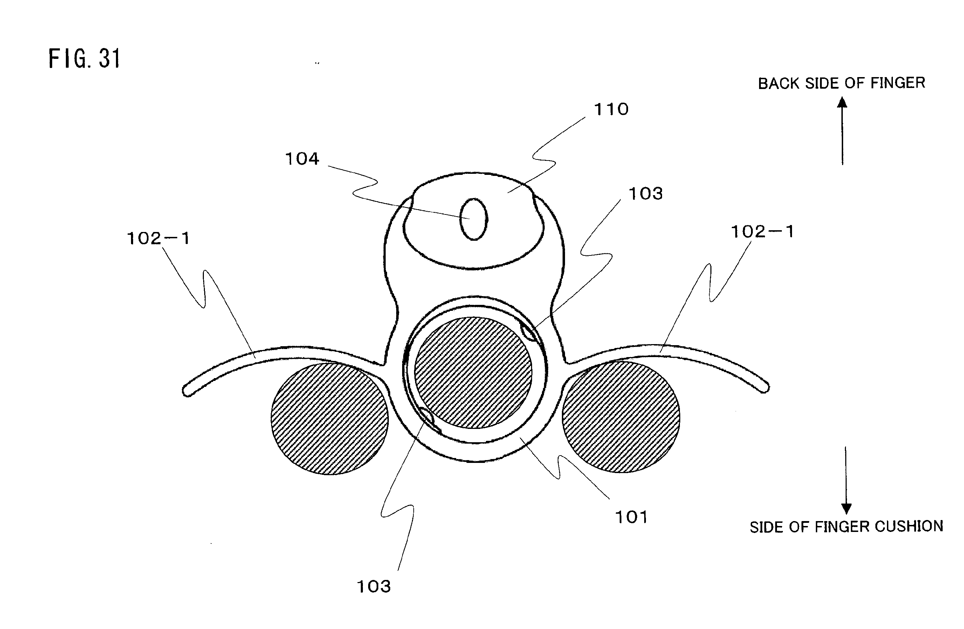

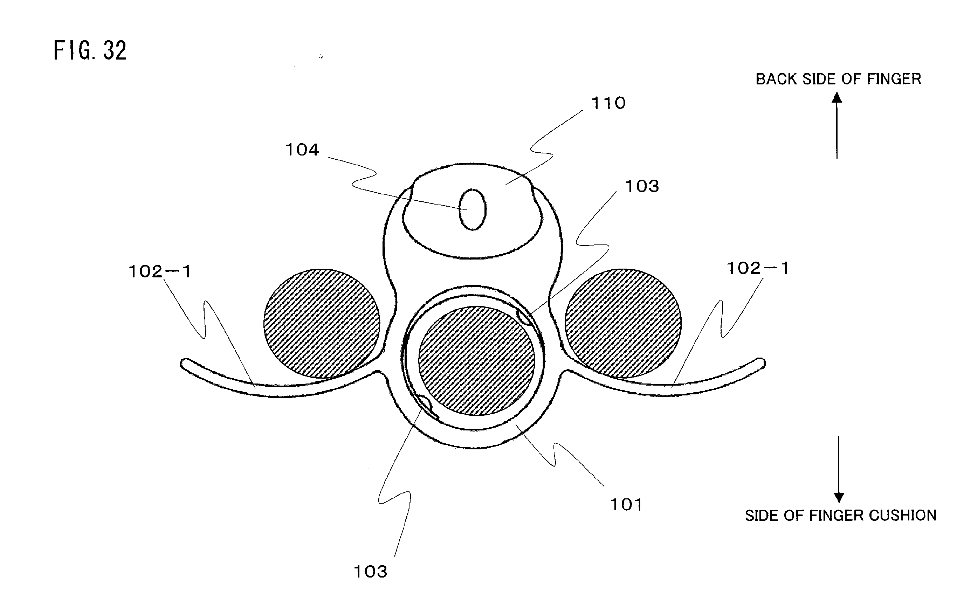

Each of Fig. 31 and Fig. 32 is a front view of wearing a biological signal measurement device of the present embodiment. Fig. 31 shows a biological signal measurement device whose measurement environment adjusting section (rotation adjusting section) 102-1 touches the back of a finger. The shaded areas in the figure are cross sections of a finger wearing the biological signal measurement device and supporting fingers. If the measurement environment adjusting section (rotation adjusting section) 102-1 is made of an elastic material so that the shape of arch can be reversed, the shape of the measurement environment adjusting section (rotation adjusting section) 102-1 will be as shown in Fig. 32. In this case, the biological signal measurement device is worn on a finger so that the finger wearing the device and supporting fingers are positioned as shown by the shaded areas of Fig. 32. In this case, the measurement environment adjusting section (rotation adjusting section) 102-1 touches the finger cushion side of the fingers. As described, with the measurement environment adjusting section (rotation adjusting section) 102-1 whose arch-shaped portion can be easily reversed, the part the measurement environment adjusting section 102-1 touches can be easily changed from the back of finger to the finger cushion or other way around. With this, the biological signal measurement device can be suitably worn and used for various usage scenes of everyday life, thus allowing highly accurate measurement.

Further, in the above mentioned case where the measurement environment adjusting section (rotation adjusting section) 102-1 is made compatible so as to touch either the back of a finger or the finger cushion, it is preferable that the biological signal measurement device include two sensing sections 103, one for each position as shown in Fig. 31 and Fig. 32. In this way, no matter which side the measurement environment adjusting section 102-1 is positioned, at least one of the sensing sections 103 is properly attached in a position of the measuring object (an artery or the like on the finger cushion side), thereby allowing most suitable measurement. Of course, the biological signal measurement device does not necessarily have to have two sensing sections 103, provided that sufficiently accurate measurement is possible with a single sensing section. That is, the biological signal measurement device does not need two sensing sections 103 as in the above case, as long as the biological signal measurement device is also capable of performing sufficiently accurate measurement with respect to a blood vessel on the backhand side of a finger (i.e., dorsal digital artery).

In the present embodiment, a measurement environment adjusting section (pressure adjustment section) 102-2 includes a gear and a projecting section 102-2A. As in Embodiment 4, it is possible to further provide a pressure detecting section 201 and a fitting adjustment drive section 102-A so that a squeezing force can be automatically adjusted. With the provision of a structure that allows the measurement environment adjusting section (pressure adjustment section) 102-2 to perform automatic adjustment, the squeezing force can be adjusted by the measurement environment adjusting section (pressure adjustment section) 102-2, based on a measurement environment having been judged by a measurement environment judging section 107, thus optimizing the measurement environment without a need of user's manual operation. Note that the adjusting mechanism is not limited to the above-described example, and any adjustment can be adopted.

Each of Embodiments 5 to 7 is suitably provided with features described in Embodiments 1 to 4. Accordingly, it is needless to say that the similar effects are obtained.

With the similar structure, the ring sensor 100 can be worn on a part of a human body. When the measurement processing section 108 performs the measurement of the biological signal, if the sensing section is not able to measure biological signal from the part of the human body under suitable conditions, the measurement environment judging section 107 judges the measurement environment obtained by one of the measurement environment sensing section 103, pressure detecting section 201, and position detecting section 202, or any combination of these. The result of judgment is notified by the measurement environment notifying section 104. Accordingly, the user is able to easily adjust the measurement environment by operating the measurement environment adjusting section 102, based on the judgment result notified by the measurement environment notifying section 104.

Further, with the provision of the measurement environment adjusting section 102 which adjust the measurement environment of the sensing section according to the judgment result obtained from the measurement environment judging section 107, the measurement environment judging section 107 judges the measurement environment obtained from the measurement environment sensing section 103 or the like, and the measurement environment adjusting section 102 adjusts the measurement environment of the sensing section based on the judgment result. Thus, the user only needs to wear the biological signal measurement device, and the suitable measurement environment is achieved without a need of the user operating the measurement environment adjusting section.

### (Common Alternative Form)

The measurement environment notifying section 104 may notify a measurement environment through the following method. For example, the measurement environment may be notified in the form of light emission from an LED and/or music from a speaker. Further, the measurement environment may be notified in the form of bar graph plotting the environment conditions as shown in Fig. 12.

There may be a case where how the user usually wears the biological signal measurement device results in a poor measurement environment due the using conditions or his/her body conditions, consequently necessitating an increase in the squeezing force applied by the fitting section 101 or more effective prevention of rotation by the measurement environment adjusting section 102. However, by notifying the measurement environment in such a manner that the user is easily able to understand, the user is able to adjust the pressure applied by the fitting section 101 to the user's finger by lengthening the measurement environment adjusting section 102, while confirming the accuracy notified by the measurement environment notifying section 104. Thus, the user is able to avoid blood congestion or the like by wearing the biological signal measurement device in a way that suits user's hand, and more highly-accurate measurement can be performed.

Next described are variations of the way by which the measurement environment notifying section 104 notifies the user of the judged accuracy. For example, the following three ways are possible.
(1) Providing an LED to the biological signal measurement device, and notify whether or not the accuracy meets the criterion by turning on/off the LED or by changing the style of emitting light.
(2) Providing a speaker to the biological signal measurement device, and notify whether or not the accuracy meets the criterion, by turning on/off the speaker or by producing various sounds or sounds at various volumes.
(3) Providing display means (e.g. liquid crystal display) to the biological signal measurement device, and notify whether or not the accuracy meets the criterion in the form of different symbols or different numbers.

In a case of (1) using the LED, the notification is made as follows. If the accuracy meets the criterion value, the LED is constantly turned on. If not, the LED is turned on/off according to how much more the accuracy needs to be improved to meet the criterion. For example, if the accuracy is just about to meet the criterion, the LED slowly blinks. On the other hand, if the accuracy is so far from clearing the criterion value due to considerably poor fitting conditions of the biological signal measurement device, the LED quickly blinks.

In a case of (2) using the speaker, the notification is made as follows. The biological signal measurement device is provided with the speaker 104-2 as shown in Fig. 11. If the accuracy did not meet the criterion, a beep sound is made to warn the user. Further, according to how much more the accuracy needs to be improved to meet the criterion, it is possible to change the tone or an interval of the beep sounds, or use different kinds of sounds: e.g. a piece of music or singing of a little bird for when the accuracy is just about to meet the criterion; and a siren of an ambulance or the like for when the accuracy is far from meeting the criterion. Further, by configuring the biological signal measurement device so that the sound is stopped by means of operating the operation button 105, it is possible to avoid making sound when it is not needed. This saves the power, and a low-power-consuming human-wearable sensor 100 can be realized.

In a case of (3) using the display means, the notification is made as follows. With a use of display means such as a liquid crystal display shown in Fig. 12, it is preferable to display, for example, symbols and/or numbers in such a manner that the user is able to understand the notification at a glance. In the example shown in Fig. 12, the dotted line is the criterion of the accuracy (i.e., if the accuracy is usable if it is above the line). The displaying is made easier for the user to understand as follows. When the accuracy is high, the number of black rectangles on the display section increases. On the contrary, if the accuracy is low, the number of black rectangles on the display section decreases. Further, if the display means also has a function of displaying the measurement biological signal in real time, the user is able to know the current conditions of him/herself. This allows the user to measure his/her biological signal in an easy-to-understand manner, and the user is able to have fun in doing so.

As described, with the provision of the measurement environment notifying section 104 such as LED, speaker, or display means, the user him/herself is able to judge the measurement environment through his/her eyes or ears, and is able to adjust the squeezing force, the length or position of rotation preventing section, for more highly accurate measurement.

Note that each measurement environment adjusting section 102 of Embodiments 1 to 7 adjusts a measurement environment of the sensing section 103 by adjusting an amount of projection from the main body 110. However, the structure of the measurement environment adjusting section 102 is not limited to this. For example, the measurement environment adjusting section 102 may have a winding-up structure or the like. Furthermore, the structure for adjusting the length of the fitting section 101 (grooves) may be provided to one of or both of the rotation preventing sections 104.

Furthermore, the respective directions and positions of the measurement environment notifying section 104, the measurement environment judging section 107, the measurement environment adjusting section 102, and the main body 110 each described in Embodiments 1 through 4, with reference to the figures are mere examples. Each Member may be arranged in any position and direction, provided that the sensing section 103 is properly arranged.

Incidentally, when a measurement of a biological signal is performed with respect to an artery according to the above-mentioned principal, possible disturbance factors other than the pressure and position are: disturbance light such as the sunlight; acceleration during the measurement; temperature (temperature of an ultra cold region, or temperature in a sauna) ; and so on. Accordingly, provision of sensors for sensing disturbance light, acceleration, and temperature, the disturbance factors can be specified and feedbacked to the user more in detail. Alternatively, by reflecting the sensed disturbance factors to the adjustment, it is possible to improve the reliability. In a case of periodically performing the measurement, provision of those sensors allows the measurement to be performed only when the measurement environment is favorable. This further unburdens the user.

Alternatively, in a case of periodically performing the measurement, if a severe change of body conditions or a sign of such a change is detected, it is possible to change the measurement process so that the measurement is performed more frequently or continuously. At the same time, it is possible to send a signal calling for a help to an external device such as a mobile phone, via the wireless-communication section 109. Alternatively, it is possible to inform the user or a person around the user that a help is needed, by means of notification made by the measurement environment notifying section 104.

Further, each of the above embodiments deals with a circular or hyperbolic human-wearable sensor 100 to be worn on a finger. However, it is needless to say that the same effect can be obtained by a human-wearable sensor designed to be worn on a toe.

Further, by forming the human-wearable sensor in a shape of a plane disc part of a wrist watch or the like and providing the measurement environment adjusting section (rotation adjusting section) in the human-wearable sensor, it is possible to realize a human-wearable sensor to be worn on a wrist. Specifically, since a radius nearby a wrist and an area nearby an ulna have thin muscle and the back of a hand normally has a relatively flat shape, rotation is prevented by closely attaching the plane disc part to the back of the hand. Further, instead of finger or wrist, the present invention can be worn on a part of a human body having a circular or hyperbolic shape (torso).

As described, a biological signal measurement device according to a first form of the present invention includes: a fitting section to be attached to a part of a human body; a sensing section, provided to the fitting section, which is to be closely attached to a specific part of the human body so as to sense the biological signal; a measurement processing section for performing a measurement of the biological signal by using the sensing section; a measurement environment judging section for judging a measurement environment based on an output of the sensing section; a measurement environment notifying section for notifying a user of a judgment result, according to a result given by the measurement environment judging section; and a measurement environment adjusting section for adjusting the measurement environment of the sensing section. It is preferable that the biological signal measurement device include: a measurement environment detecting section for detecting the measurement environment of the sensing section, wherein the measurement environment judging section judges the measurement environment based on an output of the sensing section or the measurement environment detecting section.

When the measurement processing section performs the measurement of the biological signal while the biological signal measurement device is worn on a part of a human body, and if the sensing section is not able to measure the biological signal from the part of the human body under suitable conditions, the measurement environment judging section judges the measurement environment of the sensing section which is obtained from the measurement environment detecting section. The judgment result is then notified by the measurement environment notifying section. Thus, the user is able to easily adjust the measurement environment to a favorable environment, by operating the measurement environment adjusting section, based on the judgment result of the measurement environment notified by the measurement environment notifying section.

Preferably, when a judgment of the measurement environment performed by the measurement environment judging section is unfavorable, the measurement environment notifying section periodically or continuously performs notification until a favorable measurement environment is achieved.

Since the measurement environment notifying section periodically or continuously notifies the judgment result until a favorable measurement environment is achieved, the user is able to know that the measurement environment of the sensing section is suitably adjusted, when the notification from the measurement environment notifying section stops.

Further, it is preferable that the measurement environment detecting section detect plural pieces of environment information, and that the measurement environment notifying section notify the judgment results obtained based on the plural pieces of environment information in different forms respectively. It is further preferable that (A) the measurement environment judging section make judgment taking into account at least two of (i) a measurement environment obtained based on a position of the sensing section; (ii) a measurement environment obtained based on a pressure applied by the sensing section; and (iii) a measurement environment obtained based on a biological signal and that (B) the measurement environment notifying section notify the judgment results obtained based on the plural pieces of environment information in different forms respectively.

Since the measurement environment notifying section notifies the judgment results obtained based on the plural pieces of environment information in different forms respectively, the user is able to more specifically know the measurement environment. For example, the user is able to know that the pressure is suitable but not the position. Knowing this, the user is able to figure out how to make adjustment by using the measurement environment adjusting section.

Further, a biological signal measurement device according to a second form of the present invention includes: a fitting section to be attached to a part of a human body; a sensing section, provided to the fitting section, which is to be closely attached to a specific part of the human body so as to sense the biological signal; a measurement processing section for performing a measurement of the biological signal by using the sensing section; a measurement environment detecting section for detecting the measurement environment of the sensing section; a measurement environment judging section for judging the measurement environment based on an output of the measurement environment detecting section; and a measurement environment adjusting section for adjusting the measurement environment of the sensing section according to a result given by the measurement environment judging section.

Since the measurement environment judging section judges the measurement environment of the sensing section obtained from the measurement environment detecting section, and the measurement environment adjusting section adjusts the measurement environment of the sensing section, based on the judgment result, the user only needs to wear the biological signal measurement device. The measurement environment is adjusted to a suitable environment without a need of the user operating the measurement environment adjusting section.

It is preferable that, when a judgment of the measurement environment performed by the measurement environment judging section is favorable, the measurement processing section executes the measurement of the biological signal based on an output from the sensing section.

Since the measurement processing section executes the measurement when the measurement environment is judged as to be favorable. The measurement is less likely to be failed.

It is further preferable that, when the measurement result of the measurement processing section is unfavorable, the measurement environment notifying section notifies that the measurement result is unfavorable.

Thus, when the measurement environment notifying section notifies that the measurement result of the measurement processing section is unfavorable, the user is able to know that the measurement has failed.

It is preferable that the measurement environment judging section judges the measurement environment, based on the biological signal sensed by the sensing section. For example, it is preferable that the measurement environment judging section judge the measurement environment based on an amplitude of a predetermined frequency component of the biological signal having been sensed by the sensing section.

Since an actual measurement is performed after judging whether or not a signal with a sufficient amplitude is obtained, by using the sensing section used in the actual measurement, a highly accurate measurement is possible.

It is preferable that the measurement environment detecting section detect a relative position and a pressure environment of the sensing section in relation to the part of the human body.

Thus, the measurement environment judging section is able to more specifically judge the measurement environment, based on the pressure environment or the position environment detected by the environment detecting section.

It is preferable that the measurement environment detecting section periodically detect the measurement environment; and the measurement processing section periodically perform the measurement of a biological signal.

By periodically performing the detecting process or the measurement process, it is possible to save the power and reduce the size of unnecessary data.

The measurement environment adjusting section adjusts the pressing force (pressure) applied by the sensing section to the part of the human body, or the relative position of the sensing section to the human body.

With the measurement environment adjusting section, it is possible to adjust the pressing force (pressure) applied by the detesting section or the relative position of the sensing section, each of which is a major factor affecting a measurement environment for optically sensing pulse waves, particularly from an artery.

It is further preferable that the measurement environment adjusting section is driven so as to loosen the fitting section, when detecting of the measurement environment or measuring of a biological signal is not performed.

Since the fitting section is loosened when the measurement is not performed, the user is less afflicted with numbness or feeling of tightness in the finger.

Further, the measurement environment adjusting section is capable of maintaining the measurement environment of the sensing section. For example, the measurement environment adjusting section prevents rotation of the biological signal measurement device.

Since the measurement environment adjusting section maintains the measurement environment, the measurement environment is maintained to a favorable state, once the user adjust the measurement environment to the favorable state. Thus, the user needs to adjust the environment less frequently.

A biological signal measurement method which is a third form of the present invention includes the steps of: (I) judging a measurement environment for sensing a biological signal through a close contact to a specific part of the human body; (II) giving notification to a user when the measurement environment is judged as to be unfavorable; and (III) executing a predetermined measurement of a biological signal when the measurement environment is judged as to be favorable.

It is further preferable that, if the measurement environment is not judged as to be favorable in the (I), the (I) and (II) are looped until the measurement environment is judged as to be favorable in the (I).

A biological signal measurement method which is a fourth form of the present invention includes the steps of: (I) judging a measurement environment for sensing a biological signal through a close contact to a specific part of the human body; (II) adjusting the measurement environment when the measurement environment is judged as to be unfavorable; and (III) executing a predetermined measurement of a biological signal when the measurement environment is judged as to be favorable.

It is further preferable that, if the measurement environment is not judged as to be favorable in the (I), the (I) and (II) be repeated until the measurement environment is judged as to be favorable in the (I).

A computer program which is a fifth form of the present invention is computer program for causing a computer to perform the above-described biological signal measurement method.

### INDUSTRIAL APPLICABILITY

A biological signal measurement device of the present invention can be worn on a human body for a purpose of measuring a pulse wave or blood oxygen saturation. Thus, the present invention allows a user to wear the device and easily adjust and maintain a favorable measurement conditions without a lot of work.

## Claims

1. A human-wearable biological signal measurement device which constantly or periodically measures a biological signal, comprising:
a fitting section to be attached to a part of a human body;
a sensing section, provided to the fitting section, which is to be closely attached to a specific part of the human body so as to sense the biological signal;
a measurement processing section for performing a measurement of the biological signal by using the sensing section;
a measurement environment judging section for judging a measurement environment based on an output of the sensing section;
a measurement environment notifying section for notifying a user of a judgment result, according to a result given by the measurement environment judging section; and
a measurement environment adjusting section for adjusting the measurement environment of the sensing section.

2. A human-wearable biological signal measurement device which constantly or periodically measures a biological signal, comprising:
a fitting section to be attached to a part of a human body;
a sensing section, provided to the fitting section, which is to be closely attached to a specific part of the human body so as to sense the biological signal;
a measurement processing section for performing a measurement of the biological signal by using the sensing section;
a measurement environment detecting section for sensing the measurement environment of the sensing section;
a measurement environment judging section for judging the measurement environment based on an output of the measurement environment detecting section;
a measurement environment notifying section for notifying a user of a judgment result, according to a result given by the measurement environment judging section;
a measurement environment adjusting section for adjusting the measurement environment of the sensing section;

3. The biological signal measurement device as set forth in claim 2, wherein:
the measurement environment judging section further judges the measurement environment based on an output of the sensing section.

4. The biological signal measurement device as set forth in any one of claims 1 through 3, wherein:
when a judgment of the measurement environment performed by the measurement environment judging section is unfavorable, the measurement environment notifying section periodically or continuously performs notification until a favorable measurement environment is achieved.

5. The biological signal measurement device as set forth in any one of claims 1 through 4, wherein:
when a judgment of the measurement environment performed by the measurement environment judging section is favorable, the measurement processing section executes the measurement of the biological signal based on an output from the sensing section.

6. The biological signal measurement device as set forth in any one of claims 1 through 3, wherein:
the measurement environment judging section respectively derives judgment results based on plural pieces of environment information.

7. The biological signal measurement device as set forth in claim 6, wherein:
the measurement environment notifying section notifies the judgment results obtained based on the plural pieces of environment information in different forms, respectively.

8. The biological signal measurement device as set forth in claim 6 or 7, wherein:
the measurement environment judging section makes judgment taking into account at least two of (i) a measurement environment obtained based on a position of the sensing section; (ii) a measurement environment obtained based on a pressure applied by the sensing section; and (iii) a measurement environment obtained based on a biological signal.

9. A human-wearable biological signal measurement device which constantly or periodically measures a biological signal, comprising:
a fitting section to be attached to a part of a human body;
a sensing section, provided to the fitting section, which is to be closely attached to a specific part of the human body so as to sense the biological signal;
a measurement processing section for performing measurement of the biological signal by using the sensing section;
a measurement environment detecting section for detecting the measurement environment of the sensing section;
a measurement environment judging section for judging the measurement environment based on an output of the measurement environment detecting section; and
a measurement environment adjusting section for adjusting the measurement environment of the sensing section according to a result given by the measurement environment judging section.

10. The biological signal measurement device as set forth in claim 9, wherein:
the measurement environment judging section judges the measurement environment based on an output of the measurement environment detecting section and an output of the sensing section.

11. The biological signal measurement device as set forth in claim 1, 3 or 10, wherein:
the measurement environment judging section judges the measurement environment based on an amplitude of a predetermined frequency component of the biological signal having been sensed by the sensing section.

12. The biological signal measurement device as set forth in any one of claims 9 through 11, wherein:
when a judgment of the measurement environment performed by the measurement environment judging section is favorable, the measurement processing section executes the measurement of a biological signal based on an output from the sensing section.

13. The biological signal measurement device as set forth in any one of claims 9 through 11, further comprising:
a measurement environment notifying section for making notification to the user according to a judgment result of the measurement environment judging section, wherein
when the measurement result of the measurement processing section is unfavorable, the measurement environment notifying section notifies that the measurement result is unfavorable.

14. The biological signal measurement device as set forth in any one of claims 9 through 11, wherein
the measurement environment adjusting section is driven so as to loosen the fitting section, when detecting of the measurement environment or measuring of a biological signal is not performed.

15. The biological signal measurement device as set forth in any one of claims 1 through 3, 9, and 10, wherein:
the measurement environment detecting section periodically detects the measurement environment; and
the measurement processing section periodically performs the measurement of a biological signal.

16. The biological signal measurement device as set forth in any one of claims 2 through 15, wherein:
the measurement environment detecting section detect a pressure environment of the sensing section in relation to the part of the human body.

17. The biological signal measurement device as set forth in any one of claims 2 through 15, wherein:
the measurement environment detecting section periodically detects the measurement environment; and
the measurement processing section periodically performs the measurement of a biological signal.

18. The biological signal measurement device as set forth in any one of claims 2 through 15, wherein:
the measurement environment detecting section detects a relative position of the sensing section to the part of the human body.

19. The biological signal measurement device as set forth in claim 18, wherein:
the measurement environment detecting section detects the relative position of the sensing section to the human body based on volumes of sounds detected by two or more microphones.

20. The biological signal measurement device as set forth in any one of claims 1 through 15, wherein:
the measurement environment adjusting section adjusts a pressure applied by the sensing section to the part of the human body.

21. The biological signal measurement device as set forth in any one of claims 1 through 15, wherein:
the measurement environment adjusting section adjust the relative position of the sensing section to the human body.

22. The biological signal measurement device as set forth in any one of claims 1 through 15, wherein:
the fitting section includes an anti-rotation member for preventing rotation of the biological signal measurement device about the human body.

23. The biological signal measurement device as set forth in claim 22, wherein:
the anti-rotation member for preventing rotation of the biological signal measurement device about the human body is formed in one piece with the measurement environment adjusting section.

24. A biological signal measurement method to be worn by a human body, which method constantly or periodically measures a biological signal, comprising the steps of:
(I) judging a measurement environment for sensing a biological signal through a close contact to a specific part of the human body;
(II) giving notification to a user when the measurement environment is judged as to be unfavorable; and
(III) executing a predetermined measurement of a biological signal when the measurement environment is judged as to be favorable.

25. A biological signal measurement method to be worn by a human body, which method constantly or periodically measures a biological signal, comprising the steps of:
(I) judging a measurement environment for sensing a biological signal through a close contact to a specific part of the human body;
(II) adjusting the measurement environment when the measurement environment is judged as to be unfavorable; and
(III) executing a predetermined measurement of a biological signal when the measurement environment is judged as to be favorable.

26. The method as set forth in claim 24 or 25, wherein:
if the measurement environment is not judged as to be favorable in the (I), the (I) and (II) are looped until the measurement environment is judged as to be favorable in the (I).

27. A computer program for causing a computer to perform the biological signal measurement method as set forth in any one of claims 24 to 26.

## Amended claims

### Amended claims under Art. 19.1 PCT

**1.** Amended) A biological signal measurement device to be worn on a part of a human body, for measuring a biological signal from the human body, comprising:
a sensing section for sensing the biological signal from the human body;
a measurement environment detecting section for detecting a fitting-state of the sensing section on the human body; and
a measurement environment judging section for judging whether or not the fitting-state is favorable, based on outputs of the measurement environment detecting section and the sensing section.

**2.** Amended) The biological signal measurement device as set forth in claim 1, comprising:
a measurement environment notifying section for notifying a user of a judgment result obtained from the measurement environment judging section; and
a measurement environment adjusting section for adjusting the fitting-state of the sensing section.

**3.** Amended) The biological signal measurement device as set forth in claim 1 or 2, wherein:
a measurement environment detecting section detects a measurement position of the sensing section as the fitting state.

**4.** Amended) The biological signal measurement device as set forth in Claim 3, wherein:
the sensing section senses the biological signal from an artery; and
the measurement position of the sensing section detected by the measurement environment detecting section is a position of the sensing section relative to the artery.

**5.** Amended) The biological signal measurement device as set forth in Claim 4, further comprising:
a circular or hyperbolic fitting section which allows the biological signal measurement device to be worn on the part of the human body, wherein:
the sensing section is fixed on the fitting section; and
the position relative to the artery which position is detected by the measurement environment detecting section is a position of the sensing section relative to the artery, the sensing section being arranged on a circumference of the circular or hyperbolic fitting section.

**6.** Amended) The biological signal measurement device as set forth in Claim 4 or 5, wherein:
the measurement environment detecting section detects the relative position of the sensing section to the artery, based on sound volumes detected by two microphones.

**7.** Amended) The biological signal measurement device as set forth in Claim 6, wherein:
the microphones are arranged on the circumference of the fitting section on which the sensing section is fixed, and the microphones are symmetrically arranged in relation to the sensing section.

**8.** Amended) The biological signal measurement device as set forth in any one of claims 1 through 7, wherein:
the measurement environment judging section judges whether or not an output from the measurement environment detecting section is within a range which allows a suitable measurement of the biological signal; and when the measurement environment judging section judges that the output of the measurement environment detecting section is within the suitable range, whether or not the fitting-state is favorable is determined, by judging whether or not a result of detection by the sensing section is normal.

**9.** Amended) The biological signal measurement device as set forth in Claim 8, wherein:
the measurement environment detecting section detects a pressing force applied by the sensing section to the part of the human body; and
the measurement position of the sensing section is judged as to be unfavorable, when (i) the measurement environment judging section judges that the pressing force detected by the measurement environment detecting section is within the range which allows a suitable measurement of the biological signal and (ii) the result of the detection by the sensing section is judged as to be not normal.

**10.** Amended) The biological signal measurement device as set forth in Claim 8, wherein:
the measurement environment detecting section detects plural kinds of information regarding the fitting-state; and
the measurement environment judging section judges the fitting-state of the biological signal measurement device, based on the plural kinds of information regarding the fitting-state detected.

**11.** Amended) The biological signal measurement device as set forth in Claim 10, wherein:
the plural kinds of information regarding the fitting-state at least include: the pressing force applied by the sensing section to the part of the human body and the measurement position of the sensing section.

**12.** Amended) The biological signal measurement device as set forth in Claim 9 or 11, wherein:
the measurement environment detecting section for detecting the pressing force is arranged between the fitting section and the sensing section.

**13.** Amended) The biological signal measurement device as set forth in Claim 10 or 11, wherein:
the measurement environment notifying section outputs judgment results of the plural kinds of information regarding the fitting-state in different forms, respectively.

**14.** Amended) The biological signal measurement device as set forth in any one of claims 1 through 13, further comprising:
a measurement processing section for performing the measurement of the biological signal, by controlling the sensing section so that the sensing section senses the biological signal, wherein
the measurement processing section executes the measurement of the biological signal according to the judgment results of the measurement environment judging section.

**15.** Amended) The biological signal measurement device as set forth in Claim 14, wherein:
the measurement processing section intermittently executes the measurement of the biological signal.

**16.** Amended) A biological signal measurement device to be worn on a part of a human body, for measuring a biological signal from the human body, comprising:
a sensing section, for sensing the biological signal from the human body;
a measurement environment detecting section for detecting a measurement position of the sensing section in relation to the part of the human body, or a pressing force applied by the sensing section to the part of the human body;
a measurement environment judging section for judging, based on an output of the measurement environment detecting section, whether or not the measurement position of the sensing section or the pressing force applied by the sensing section is within a range which allows a suitable measurement of the biological signal; and
a measurement environment adjusting section for adjusting the measurement position of the sensing section or the pressing force applied by the sensing section, according to a judgment result of the measurement environment judging section.

**17.** Amended) The biological signal measurement device as set forth in Claim 16, wherein:
when measurement environment judging section judges that the pressing force is within the range which allows a suitable measurement of the biological signal, the measurement environment adjusting section adjusts the measurement position of the sensing section until a predetermined of amplitude of a predetermined frequency component is obtained.

**18.** Amended) The biological signal measurement device as set forth in Claim 16 or 17, further comprising:
a fitting section by which the biological signal measurement device is worn on the part of the human body, wherein:
the sensing section is fixed on the fitting section;
the measurement environment detecting section includes a pressing force detecting section for detecting the pressing force applied by the sensing section to the part of the human body, the pressing force detecting section being arranged between the fitting section and the sensing section.

**19.** Amended) The biological signal measurement device as set forth in any one of claims 16 through 18, wherein:
the sensing section senses the biological signal from an artery; and
the measurement position of the sensing section detected by the measurement environment detecting section is a relative position of the sensing section to the artery.

**20.** Amended) The biological signal measurement device as set forth in Claim 19, further comprising:
a circular or hyperbolic fitting section which allows the biological signal measurement device to be worn on the part of the human body, wherein:
the sensing section is fixed on the fitting section; and
the position relative to the artery which position is detected by the measurement environment detecting section is a position of the sensing section relative to the artery, the sensing section being arranged on a circumference of the circular or hyperbolic fitting section.

**21.** Amended) The biological signal measurement device as set forth in Claim 19 or 20, wherein:
the measurement environment detecting section detects the relative position of the sensing section to the artery, based on sound volumes detected by two or more microphones.

**22.** Amended) The biological signal measurement device as set forth in Claim 21, wherein:
the microphones are arranged on the circumference of the fitting section on which the sensing section is fixed, and the microphones are symmetrically arranged in relation to the sensing section.

**23.** Amended) The biological signal measurement device as set forth in any one of claims 20 through 22, further comprising:
an anti-rotation member for preventing the circular or hyperbolic fitting section from rotating about the part of the human body.

**24.** Amended) The biological signal measurement device as set forth in any one of claims 16 through 23, further comprising:
a measurement environment notifying section for notifying a user of the judgment result of the measurement environment judging section.

**25.** Amended) A method of operating a biological signal measurement device which is worn on a part of a human body, and which measures a biological signal from the human body, comprising the steps of:
(I) detecting a fitting-state at a time of sensing the biological signal from the human body;
(II) sensing the biological signal from the human body; and
(III) judging whether or not the fitting-state is favorable, based on fitting-state detected in (I) and the biological signal detected in (II).

**26.** Amended) A method of operating a biological signal measurement device which is worn on a part of a human body, and which measures a biological signal from the human body, comprising the steps of:
(I) detecting a measurement position in relation to the human body or a pressing force applied to the human body, at a time of sensing the biological signal from the human body;
(II) judging whether or not the measurement position or the pressing force detected in (I) is within a range which allows a suitable measurement of the biological signal; and
(III) adjusting the measurement position or the pressing force according to the result of judgment in (II).

**27.** Amended) A computer program for executing the method as set forth in Claim 25 or 26, which causes a computer to execute each of the steps as set forth hereinabove.
